(19) Europäisches Patentamt
European Patent Office
Office européen des brevets

(11) EP 1 603 921 B1

(12) **EUROPEAN PATENT SPECIFICATION**

(45) Date of publication and mention of the grant of the patent:
**17.06.2009 Bulletin 2009/25**

(21) Application number: **04717708.4**

(22) Date of filing: **05.03.2004**

(51) Int Cl.:
*C07D 495/04* (2006.01) *A61K 31/382* (2006.01)
*A61P 29/00* (2006.01)

(86) International application number:
**PCT/HR2004/000005**

(87) International publication number:
**WO 2004/078763 (16.09.2004 Gazette 2004/38)**

(54) **THIADIBENZOAZULENE DERIVATIVES FOR THE TREATMENT OF INFLAMMATORY DISEASES**
THIADIBENZOAZULENDERIVATE ZUR BEHANDLUNG VON ENTZÜNDLICHEN ERKRANKUNGEN
DERIVES DE THIADIBENZOAZULENE POUR LE TRAITEMENT DE MALADIES INFLAMMATOIRES

(84) Designated Contracting States:
**AT BE BG CH CY CZ DE DK EE ES FI FR GB GR HU IE IT LI LU MC NL PL PT RO SE SI SK TR**
Designated Extension States:
**AL LT LV MK**

(30) Priority: **06.03.2003 HR 20030160**

(43) Date of publication of application:
**14.12.2005 Bulletin 2005/50**

(73) Proprietor: **GlaxoSmithKline istrazivacki centar Zagreb d.o.o.**
**10000 Zagreb (HR)**

(72) Inventors:
- **MERCEP, Mladen**
  **HR-10000 Zagreb (HR)**
- **MESIC,Milan**
  **HR-10000 Zagreb (HR)**
- **PESIC, Dijana**
  **HR-22000 Sibenik (HR)**
- **BENKO, Iva**
  **HR-10410 Velika Gorica (HR)**

(74) Representative: **von Füner, Nicolai et al**
**v. Füner Ebbinghaus Finck Hano**
**Patentanwälte**
**Mariahilfplatz 2&3**
**81541 München (DE)**

(56) References cited:
**WO-A-01/87890**
**WO-A-03/097649**
**US-A- 3 711 489**
**US-A- 4 198 421**
**US-A- 5 917 057**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to (a) novel 1-thiadibenzoazulene derivatives of the formula (I), (b) to their pharmacologically acceptable esters, salts and solvates, (c) to processes and intermediates for the preparation thereof, (d) to a process for preparing pharmaceutical formulations for the treatment of inflammatory diseases and conditions and (e) to the use thereof in the treatment of inflammatory diseases and conditions in humans and animals. These compounds inhibit the tumour necrosis factor-α (TNP-α) production and interleukin-I (IL-1) production and exhibit an analgetic action.

Some 1,3-diaza-dibenzoazulene derivatives and salts thereof are well known as a novel class of compounds having an antiinflammatory action (US 3,711,489, US 4,198,421, CA 967,573 and HR Patent Application No. 20020453A). In the literature, from the class of 1-thia-dibenzoazulenes there are disclosed derivatives substituted in 2-position with methyl, methyl-ketone, nitro group for with carboxylic group derivatives (Cagniant PG, C. R. Hebd. Sceances Acad. Sci., 1976, 283:683-686), or with alkyloxy groups (WO 01/878990), which also possess a strong antiinflammatory action. However, according to our knowledge and to available literature data, 1-thia-dibenzoazulenes of the present invention having aminoalkyloxy substituents on benzene rings are not known. It is not known either that such compounds could possess an antiinflammatory action as inhibitors of TNF-α secretion and inhibitors of IL-1 secretion or an analgetic action.

In 1975 TNF-α was defined as a serum factor induced by endotoxin and causing tumour necrosis *in vitro* and *in vivo* (Carswell EA et al., Proc. Natl. Acad. Sci. U. S.A., 1975, 72:3666-3670). Besides antitumour action, TNF-α also exhibits numerous other biological actions important in the homeostasis of organisms and in pathophysiological conditions. The main sources of TNF-α are monocytes-macrophages, T-lymphocytes and mastocytes.

The discovery that anti-TNF-α antibodies (cA2) have an action in treating patients with rheumatoid arthritis (RA) (Elliott M et al., Lancet, 1994, 344:1105-1110) led to an increased interest in finding novel TNF-α inhibitors as possible potent drugs for RA. Rheumatoid arthritis is an autoimmune chronic inflammatory disease characterized by irreversible pathological changes in the joints. Besides in RA, TNF-α antagonists may also be used in numerous pathological conditions and diseases such as spondylitis, osteoarthritis, gout and other arthritic conditions, sepsis, septic shock, toxic shock syndrom, atopic dermatitis, contact dermatitis, psoriasis, glomerulonephritis, lupus erythematosus, scleroderma, asthma, cachexia, chronic obstructive lung disease, congestive cardiac arrest, insulin resistance, lung fibrosis, multiple sclerosis, Crohn's disease, ulcerative colitis, viral infections and AIDS.

Evidence for the biological importance of TNF-α was obtained by *in vivo* experiments in mice, which are deficient in a gen for TNF-α or its receptor. Such animals do not develop a collagen-induced arthritis model (Mori L et al., J. Immunol., 1996, 157:3178-3182) nor an endotoxin-caused septic shock condition (Pfeffer K et al., Cell, 1993, 73:457-467). Animals having an increased TNF-α level fall sick with a chronic inflammatory polyarthritis (Georgopoulos S et al., J.Inflamm., 1996, 46:86-97; Keffer J et al., EMBO J., 1991, 10:4025-4031). The pathological clinical picture of such animals is alleviated by inhibitors of TNF-α production. The treatment of such inflammatory and pathological conditions usually includes the application of non-steroid antiinflammatory drugs and, in more severe cases, gold salts, D-penicillamine or methotrexate are administered. Said drugs act symptomatically, but they do not stop the pathological process. Novel approaches in the therapy of rheumatoid arthritis are based upon drugs such as tenidap, leflunomide, cyclosporin, FK-506 and upon biomolecules neutralizing the TNF-α action. At present there are commercially available ethanercept (Enbrel, Immunex/Wyeth), a fusion protein of the soluble TNF-α receptor, and infliximab (Remicade, Centocor), a chimeric monoclonal human and mouse antibody. Besides in RA therapy, ethanercept and infliximab are also registered for the therapy of Crohn's disease (Exp. Opin. Invest. Drugs, 2000, 9:103).

In RA therapy, besides inhibition of TNF-α secretion, also the inhibition of IL-1 secretion is very important since IL-1 is an important cytokin in cell regulation and immunoregulation as well as in pathophysiological inflammatory conditions (Dinarello CA et al., Rev. Infect. Disease, 1984, 6:51). Well-known biological activities of IL-1 are: the activation of T-cells, the induction of elevated temperature, the stimulation of the secretion of prostaglandine or collagenase, the chemotaxia of neutrophils and the reduction of iron level in plasma (Dinarello CA, J. Clinical Immunology, 1985, 5:287). Two receptors to which IL-1 may bind are well known: IL-1RI and IL-1RII. By binding IL-1, IL-1RI transfers a signal intracellularly, whereas IL-1RII, also situated on the cell surface, is not able to do so. Since IL1-RII binds IL-1 as well as IL-1RI, it acts as a negative regulator of IL-1 action. Besides this mechanism of signal transfer regulation, the presence of a natural antagonist of IL-1 receptor (IL-Ira) is proven. This protein binds to IL-1RI but without any possibility of transferring any signal inside the cell. However, IL-1ra has an effect on the breaking of the signal through IL-1RI only if it is present in a concentration 500 times higher than that of IL-1. Recombinant human IL-1ra (Amgen) was clinically tested (Bresnihan B et al., Arthrit. Rheum., 1996, 39:73) and the obtained results indicated an improvement of the clinical picture over a placebo in 472 RA patients. These results indicate the importance of the inhibition of IL-1 action in treating diseases such as RA where IL-1 production is increased. Since there exists a synergistic action of TNF-α and IL-1, novel 1-thia-dibenzoazulene derivatives may be used in treating conditions and diseases related to an enhanced secretion of TNF-α and IL-1.

The present invention relates to novel 1-thia-dibenzoazulene derivatives of the formula I:

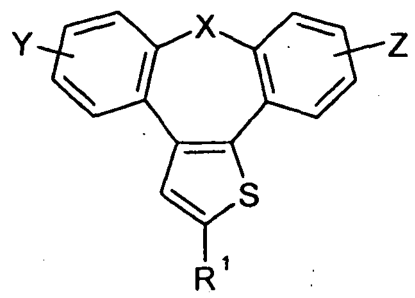

I

wherein
X individually denotes a hetero atom -O-;
Y has the meaning of H;
Z has the meaning of a fragment of the formula II:

$$-O-(CH_2)_m-A \qquad \textbf{II}$$

wherein

A individually denotes an $C_1$-$C_7$ alkyl which is substituted by 1 to 3 groups selected from halo, hydroxy, $C_1$-$C_4$ alkoxy, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; $C_2$-$C_7$ alkenyl which is unsubstituted or is substituted by 1 to 3 halo atoms; $C_2$-$C_7$ alkinyl; aryl containing one ring with at least 6 carbon atoms or two rings with totally 10 carbon atoms and with alternating double (resonant) bonds between carbon atoms, wherein the aryl group is unsubstituted or substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; $C_4$-$C_{12}$ monocyclic or bicyclic heteroaryl ring system which is aromatic or partially aromatic containing at least one heteroatom selected from oxygen, sulphur and nitrogen, wherein $C_4$-$C_{12}$ monocyclic or bicyclic heteroaryl ring system is unsubstituted or is substituted by 1 or 2 substituents selected from halo, $C_1$-$C_4$ alkyl, trifluoromethyl, cyano, nitro, hydroxy, Ci-C4 alkoxy, $C_1$-$C_4$ alkyloxycarbonyl, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; 5-6 membered heterocyclic group containing at least 1 heteroatom selected from oxygen, sulphur and nitrogen, wherein the 5-6 membered heterocyclic group is fully saturated or partially unsaturated and is unsubstituted or is substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; amino; *N*-($C_1$-$C_7$-alkyl)amino; or *N,N*-di ($C_1$-$C_7$-alkyl)amino group;

m denotes an integer from 1 to 4;

$R^1$ individually denotes hydrogen, halo, $C_1$-$C_7$ alkyl which is unsubstituted or is substituted by 1 to 3 groups selected from halo, hydroxy, $C_1$-$C_4$ alkoxy, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; $C_2$-$C_7$ alkenyl which is unsubstituted or is substituted by 1 to 3 halo atoms; $C_2$-$C_7$ alkinyl; aryl containing one ring with at least 6 carbon atoms or two rings with totally 10 carbon atoms and with alternating double (resonant) bonds between carbon atoms, wherein the aryl group is unsubstituted or is substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio, amino, *N*-($C_1$-$C_4$) alkylamino, *N,N*-di ($C_1$-$C_4$-alkyl)-amino, sulfonyl, $C_1$-$C_4$ alkylsulfonyl, sulfinyl and $C_1$-$C_4$ alkylsulfinyl; $C_4$-$C_{12}$ monocyclic or bicyclic heteroaryl ring system which is aromatic or partially aromatic containing at least one heteroatom selected from oxygen, sulphur and nitrogen, wherein $C_4$-$C_{12}$ monocyclic or bicyclic heteroaryl ring system is unsubstituted or is substituted by I or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; 5-6 membered heterocyclic group containing at least I heteroatom selected from oxygen, sulphur and nitrogen, wherein the 5-6 membered heterocyclic group is fully saturated or partially unsaturated and is unsubstituted or is substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio,

amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; hydroxy; hydroxy-$C_2$-$C_7$ alkenyl; hydroxy-$C_2$-$C_7$ alkinyl; $C_1$-$C_7$ alkoxy; thiol; thio-$C_2$-$C_7$ alkenyl; thio-$C_2$-$C_7$ alkinyl; $C_1$-$C_7$ alkylthiol; amino; N-($C_1$-$C_7$-alkyl)amino; *NN*-di($C_1$-$C_7$-alkyl)amino; $C_1$-$C_7$ alkylamino; amino-$C_2$-$C_7$ alkenyl; amino-$C_2$-$C_7$ alkinyl; amino-$C_1$-$C_7$ alkoxy; $C_1$-$C_7$ alkanoyl; aroyl, oxo-$C_1$-$C_7$ alkyl; $C_1$-$C_7$ alkanoyloxy; carboxy; $C_1$-$C_7$ alkyloxycarbonyl wherein alkyl has the meaning as defined abovearyloxycarbonyl, wherein aryl has the meaning as defined above; carbamoyl;, *N*-($C_1$-$C_7$-alkyl)carbamoyl;, *N,N*-di($C_1$-$C_7$-alkyl)carbamoyl; cyano; cyano-$C_1$-$C_7$ alkyl; sulfonyl; $C_1$-$C_7$ alkylsulfonyl; sulfinyl; $C_1$-$C_7$ alkylsulfinyl; nitro ;or a subtituent of the formula **II**, wherein the symbols A and m have the meaning as defined above;

and pharmacologically acceptable salts and solvates thereof.

The present invention relates also to a process for preparing pharmaceutical formulations containing one or more above-mentioned compounds in an amount effective for the treatment of inflammatory diseases and conditions related to an enhanced secretion of TNF-$\alpha$ and IL-1 in humans and animals.

If not stated otherwise, the following terms have the below meanings.

The term "halo" relates to a halo atom, which may be fluorine, chlorine, bromine or iodine.

The term "alkyl" relates to alkyl groups with the meaning of alkanes, wherefrom radicals are derived, which radicals may be straight, branched or cyclic or a combination of straight and cyclic ones and of branched and cyclic ones. The preferred straight or branched alkyls are e.g. methyl, ethyl, propyl, isopropyl, butyl, sec-butyl and *tert*-butyl. The preferred cyclic alkyls are e.g. cyclopentyl or cyclohexyl. Alkyl may be optionally additionally substituted with one, two or three substituents. Such substituents may be halo atom (preferably fluorine or chlorine), hydroxy, $C_1$-$C_4$ alkoxy (preferably methoxy or ethoxy), thiol, $C_1$-$C_4$ alkylthio (preferably methylthio or ethylthio), amino, *N*-($C_1$-$C_4$) alkylamino (preferably *N*-methylamino or *N*-ethylamino), *N,N*-di($C_1$-$C_4$-alkyl)-amino (preferably dimethylamino or diethylamino), sulfonyl, $C_1$-$C_4$ alkylsulfonyl (preferably methylsulfonyl or ethylsulfonyl), sulfinyl, $C_1$-$C_4$ alkylsulfinyl (preferably methylsulfinyl).

The term "alkenyl" relates to alkenyl groups having the meaning of hydrocarbon radicals, which may be straight, branched or cyclic or are a combination of straight and cyclic ones or of branched and cyclic ones, but having at least one carbon-carbon double bond. The most frequent alkenyls are ethenyl, propenyl, butenyl or cyclohexenyl. Alkenyl may be optionally additionally substituted with one, two or three halo atoms. Such substituents may be e.g. 2-chloroethenyl, 1,2-dichloroethenyl or 2-bromo-propen-1-yl.

The term "alkinyl" relates to alkinyl groups having the meaning of hydrocarbon radicals, which are straight or branched and contain at least one and at most two carbon-carbon triple bonds. The most frequent alkinyls are e.g. ethinyl, propinyl or butinyl.

The term "alkoxy" relates to straight or branched chains of alkoxy group. Examples of such groups are methoxy, propoxy, prop-2-oxy, butoxy, but-2-oxy or methylprop-2-oxy.

The term "aryl" relates to groups having the meaning of an aromatic ring, e.g. phenyl, as well as to fused aromatic rings. Aryl contains one ring with at least 6 carbon atoms or two rings with totally 10 carbon atoms and with alternating double (resonant) bonds between carbon atoms. The most freqently used aryls are e.g. phenyl or naphthyl. In general, aryl groups may be linked to the rest of the molecule by any available carbon atom via a direct bond or via a $C_1$-$C_4$ alkylene group such as methylene or ethylene. Under the term aryl there is also understood a phenyl ring fused with an optionally substituted cycloalkyl, most frequently with cyclohexane.

The term "heteroaryl" relates to groups having the meaning of aromatic and partially aromatic groups of a monocyclic or bicyclic ring with 4 to 12 carbon atoms, at least one of them being a hetero atom such as O, S or N, and the available nitrogen atom or carbon atom is the binding site of the group to the rest of the molecule. Examples of this type are thiophenyl, pyrrolyl, imidazolyl, pyridinyl, oxazolyl, thiazolyl, pyrazolyl, tetrazolyl, pirimidinyl, pyrazinyl, quinolinyl or triazinyl.

The term "heterocycle" relates to five-membered or six-membered, fully saturated or partly unsaturated heterocyclic groups containing at least one hetero atom such as O, S or N, and the available nitrogen atom or carbon atom is the binding site of the group to the rest of the molecule. The most frequent examples are morpholinyl, piperidinyl, piperazinyl, pyrrolidinyl, pyrazinyl or imidazolyl.

The term "alkanoyl" group relates to straight chains of acyl group such as formyl, acetyl or propanoyl.

The term "aroyl" group relates to aromatic acyl groups such as benzoyl.

Aryl, heteroaryl or heterocycle may be optionally additionally substituted with one or two substituents. The substituents may be halo (chlorine or fluorine), $C_1$-$C_4$ alkyl (preferably methyl, ethyl or isopropyl), trifluoromethyl, cyano, nitro, hydroxy, $C_1$-$C_4$ alkoxy (preferably methoxy or ethoxy), $C_1$-$C_4$ alkyloxycarbonyl (preferably methyloxycarbonyl) thiol, $C_1$-$C_4$ alkylthio (preferably methylthio or ethylthio), amino, *N*-($C_1$-$C_4$) alkylamino (preferably N-methylamino or *N*-ethylamino), *N,N-di($C_1$-$C_4$*-alkyl)-amino (preferably *N,N*-dimethylamino or *N,N*-diethylamino), sulfonyl, $C_1$-$C_4$ alkylsulfonyl (preferably methylsulfonyl or ethylsulfonyl), sulfinyl, $C_1$-$C_4$ alkylsulfinyl (preferably methylsulfinyl).

The term "pharmaceutically suitable salts" relates to salts of the compounds of the formula **I** (including also

quaternary ammonium salts with $C_1$-$C_4$ alkylhalides, preferably with methyl bromide and methyl chloride) with inorganic acids (hydrochloric, hydrobromic, phosphoric, metaphosphoric, nitric or sulfuric acids) or with organic acids (tartaric, acetic, citric, maleic, lactic, fumaric, benzoic, succinic, methane sulfonic or p-toluene sulfonic acids).

Some compounds of the formula **I** may form salts with organic or inorganic acids or bases and these are also included in the present invention.

Solvates (most frequently hydrates) which may be formed by compounds of the formula I or salts thereof are also an object of the present invention.

The present invention also relates to esters (e.g. acetate, formiate and benzoate derivatives) of the compounds of the formula **I.**

Depending upon the nature of particular substituents, the compounds of the formula **I** may have geometric isomers and one or more chiral centres so that there can exist enantiomers or diastereoisomers. The present invention also relates to such individual isomers and mixtures thereof including racemates. Methods for determining stereochemistry and for the resolution of stereoisomers are well known from the literature.

The present invention also relates to all possible tautomeric forms of particular compounds of the formula **I**.

In the preparation of the compounds of a specific pharmacological activity represented by the structure I, also certain novel compounds are prepared as intermediates in the preparation of pharmacologically active compounds. The present invention also relates to such intermediates.

"Pharmaceutically acceptable carrier" relates to a carrier which is used in the preparation of a pharmaceutical formulation that is generally safe, non-toxic and biologically acceptable and includes a carrier which is acceptable for pharmaceutical use in humans and animals. "Pharmaceutically acceptable carrier" in the present invention includes one or more such carriers.

A further object of the present invention relates to the preparation of compounds of the formula I according to processes comprising

a) a reaction of condensation of alcohols of the formula **IIIb:**

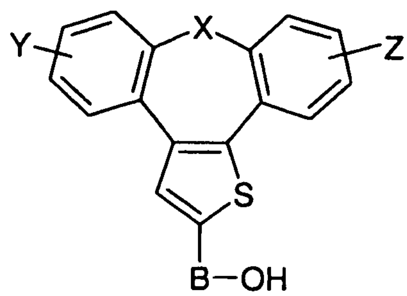

**IIIb**

wherein B individually denotes a -C(O)- or $-CH_2-$ group and Y and Z have the meaning as in the formula I, with compounds of the formula IVa:

$L^1-(CH_2)_m-A$ **IVa**

wherein $L^1$ has the meaning of a leaving group and m and A have the meaning as previously defined; or

b) a reaction of condensation of the compounds of the formula **IIIc**:

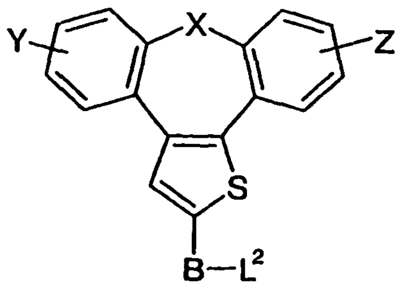

**IIIc**

wherein B individually denotes a -C(O)- or $-CH_2-$ group, $L^2$ has the individual meaning of a leaving group and Y and Z have a meaning as in the formula I,
with compounds of the formula **IVb**:

$HO-(CH_2)_m-A$ **IVb**

wherein A and m have the meaning as previously defined.

Preparation methods:

a) Compounds of the formula **I** may be prepared according to the present process by a reaction of compounds of the formula **IIIb** and of compounds of the formula **IVa,** wherein $L^1$ has the meaning of a leaving group, which may be a halo atom (most frequently bromine, chlorine or iodine) or sulfonyloxy group (most frequently trifluoromethylsulfonyloxy or p-toluenesulfonyloxy). The reactions of condensation may be carried out according to methods disclosed for the preparation of analogous compounds (Menozzi G, J. Heterocyclic Chem., 1997, 34:963-968; WO 01/87890; Jones DC et al., J. Med Chem.; 1984, 27.1057-1066). The reactions are carried out at a temperature from 20°C to 110°C, preferably 100°C, during 1 to 24 hours in a two-phase system (preferably with 50% NaOH/toluene) in the presence of a phase transfer catalyst (preferably benzyl triethyl ammonium chloride, benzyl triethyl ammonium bromide, cetyl trimethyl bromide) or under heating for several hours in *N,N*-dimethylformamide in the presence of a base (e.g. potassium carbonate). After treating the reaction mixture, the products formed are isolated by recrystallization or chromatography on a silica gel column.
The starting compounds of the formula **IIIb** are already known or are prepared according to methods disclosed for the preparation of analogous compounds (WO 01/878990).
Halides of the formula **IVa** are already known.
b) Compounds of the formula **I** according to the present process may be prepared by reacting compounds of the formula **IIIc,** wherein $L^2$ has the meaning of a leaving group defined earlier for $L^1$, and compounds of the formula **IVb.** The most suitable condensation reactions are reactions of nucleophilic substitution on a saturated and carbonyl carbon atom as disclosed in the literature.

The starting compounds of the formula **IIIc** (most frequently halides) may be obtained by halogenation (e.g. bromination of chlorination) of the compounds of the formula **IIIb** with common halogenating agents (hydrobromic acid, $PBr_3$, $SOCl_2$ or $PCl_5$) according to processes disclosed in the literature. The obtained compounds may be isolated or may be used without isolation as suitable intermediates for the preparation of the compounds of the formula **I.**
Besides the reactions mentioned above, the compounds of the formula **I** may be prepared by transforming other compounds of the formula **I** and it is to be understood that the present invention also comprises such compounds and processes.
The formylation of the compounds of the formula **I** by processes such as e.g. Vilsmeier acylation or the reaction of n-BuLi and N,N-dimethylformamide is a further general example of transformation. The reaction conditions of these processes are well known in the literature.
By hydrolysis of the compounds of the formula **I** having nitrile, amide or ester groups, there may be prepared compounds with a carboxyl group, which are suitable intermediates for the preparation of other compounds with novel

functional groups such as e.g. esters, amides, halides, anhydrides, alcohols or amines.

Oxidation or reduction reactions are a further possibility of the change of substituents in the compounds of the formula **I.** Most frequently used oxidation agents are peroxides (hydrogen peroxide, *m*-chloroperbenzoic acid or benzoyl peroxide) or permanganate, chromate or perchlorate ions. Thus e.g. by the oxidation of an alcohol group by pyridinyl dichromate or pyridinyl chlorochromate, an aldehyde group is formed, which group may be converted to a carboxyl group by further oxidation.

By a selective oxidation of an alkylthio group, alkylsulfinyl or alkylsulfonyl groups may be prepared.

By the reduction of compounds with a nitro group, the preparation of amino compounds is made possible. The reaction is carried out under usual conditions of catalytic hydrogenation or electrochemically. By catalytic hydrogenation using palladium on carbon, alkenyl substituents may be converted to alkyl ones or nitrile group can be converted to alkylamine.

Various substituents of the aromatic structure in the compounds of the formula **I** may be introduced by standard substitution reactions or by usual changes of individual functional groups. Examples of such reactions are aromatic substitutions, alkylations, halogenation, hydroxylation as well as oxidation or reduction of substituents.

Reagents and reaction conditions are known from the literature. Thus e.g. by aromatic substitution a nitro group is introduced in the presence of concentrated nitric acid and sulfuric acid. By using acyl halides or alkyl halides, the introduction of acyl groups or alkyl groups is made possible. Such reactions are carried out in the presence of Lewis acids such as aluminum- or iron-trichloride in conditions of Friedel-Crafts reaction. By the reduction of the nitro group, an amino group is obtained, which is by the reaction of diazotizing converted to a suitable starting group, which may be replaced with one of the following groups: H, CN, OH or halo. The reduction of a nitro group of aromatic compounds may be carried out in the presence of reduction agents such as Zn, Sn or Fe, or by catalytic hydrogenation.

In order to prevent undesired interaction in chemical reactions, it is often necessary to protect certain groups such as e.g. hydroxy, amino, thio or carboxy. For this purpose a great number of protecting groups may be used (Green TW, Wuts PGH, Protective Groups in Organic Synthesis, John Wiley and Sons, 1999) and the choice, use and elimination thereof are conventional methods in chemical synthesis.

A convenient protection for amino or alkylamino groups are groups such as e.g. alkanoyl (acetyl), alkoxycarbonyl (methoxycarbonyl, ethoxycarbonyl or *tert*-butoxycarbonyl); arylalkyloxycarbonyl (benzyloxycarbonyl), aroyl (benzoyl) or alkylsilyl (trimethylsilyl or trimethylsilylethoxymethyl) groups. The conditions of removing a protecting group depend upon the choice and the characteristics of this group. Thus e.g. acyl groups such as alkanoyl, alkoxycarbonyl or aroyl may be eliminated by hydrolysis in the presence of a base (sodium hydroxide or potassium hydroxide), tert-butoxycarbonyl or alkylsilyl (trimethylsilyl) may be eliminated by treatment with a suitable acid (hydrochloric, sulfuric, phosphoric or trifluoroacetic acid), whereas arylmethoxycarbonyl group (benzyloxycarbonyl) may be eliminated by hydrogenation using a catalyst such as palladium on carbon.

The most frequent protection for a hydroxy group is acetyl, benzoyl or benzyl.

Salts of the compounds of the formula I may be prepared by generally known processes such as e.g. by reacting the compounds of the formula I with a corresponding base or acid in an appropriate solvent or solvent mixture e.g. ethers (diethylether) or alcohols (ethanol, propanol or isopropanol).

Another object of the present invention concerns the use of the present compounds in the preparation of a medicament for the therapy of inflammatory diseases and conditions, especially all diseases and conditions induced by excessive TNF-$\alpha$ and IL-1 secretion.

The inhibitors of production of cytokins or inflammation mediators, which are the object of the present invention, or pharmacologically acceptable salts thereof may be used in the production of drugs for the treatment and prophylaxis of any pathological condition or disease induced by excessive unregulated production of cytokins or inflammation mediators, which drugs should contain an effective dose of said inhibitors.

The present invention specifically relates to an effective dose of TNF-$\alpha$ inhibitor, which may be determined by usual methods.

Further, the present invention relates to a pharmaceutical formulation containing an effective non-toxic dosis of the present compounds as well as pharmaceutically acceptable carriers or solvents.

The preparation of pharmaceutical formulations may include blending, granulating, tabletting and dissolving the ingredients. Pharmaceutically acceptable carriers (binders and fillers) may be solid or liquid. Solid carriers may be lactose, sucrose, talcum, gelatine, agar, pectin, magnesium stearate, fatty acids. Liquid carriers may be syrups, oils such as olive oil, sunflower oil or soya bean oil, water. Similarly, the pharmaceutically acceptable formulations may also contain a component for a sustained release of the active component such as e.g. glyceryl monostearate or glyceryl distearate. Various forms of pharmaceutical formulations may be used. Thus, if a solid carrier is used, these forms may be tablets, hard gelatine capsules, powder or granules, which may be administered in capsules per os. The amount of the solid carrier may vary, but it is mainly from 25 mg to 1 g. If a liquid carrier is used, the formulation would be in the form of a syrup, emulsion, soft gelatine capsules, sterile injectable liquids such as ampoules or non-aqueous liquid suspensions.

The compounds of the present invention may be applied per os, parenterally, locally, intranasally, intrarectally and intravaginally. The parenteral route herein means intravenous, intramuscular and subcutaneous applications. Appropriate formulations of the present compounds may be used in the prophylaxis as well as in the treatment of various diseases and conditions induced by an excessive unregulated production of cytokins or inflammation mediators, primarily TNF-α. They comprise e.g. rheumatoid arthritis, rheumatoid spondylitis, osteoarthritis and other arthritic pathological conditions and diseases, eczemas, psoriasis and other inflammatory skin conditions such as burns caused by UV radiation (sun rays and similar UV sources), inflammatory eye diseases, Crohn's disease, ulcerative colitis and asthma.

The inhibitory action of the present compounds upon TNF-α and IL-1 secretion was determined by the following *in vitro* and *in vivo* experiments:

**Determination of TNF-α and IL-1 secretion in human peripheral blood mononuclear cells *in vitro***

Human peripheral blood mononuclear cells (PBMC) were prepared from heparinized whole blood after separating PBMC on Ficoll-Paque™Plus (Amersham-Pharmacia). To determine the TNF-α level, 3.5-5x$10^4$ cells were cultivated in a total volume of 200 μl for 18 to 24 hours on microtitre plates with a flat bottom (96 wells, Falcon) in RPMI 1640 medium, into which there were added 10% FBS (Fetal Bovine Serum, Biowhittaker) previously inactivated at 54°C/30 min, 100 units/ml of penicillin, 100 mg/ml of streptomycin and 20 mM HEPES (GIBCO). The cells were incubated at 37°C in an atmosphere with 5% $CO_2$ and 90% humidity. In a negative control the cells were cultivated only in the medium (NC), whereas in a positive control TNF-α secretion was triggered by adding 1 ng/ml of lipopolysaccharides (LPS, *E. coli* serotype 0111:B4, SIGMA) (PC). The effect of the tested substances upon TNF-α secretion was investigated after adding them into cultures of cells stimulated by LPS (TS). The TNF-α level in the cell supernatant was determined by ELISA procedure according to the suggestions of the producer (R&D Systems). The test sensitivity was <3pg/ml TNF-α. The IL-1 level was determined in an assay under the same conditions and with the same number of cells and the same concentration of the stimulus by ELISA procedure (R&D Systems). The percentage of inhibition of TNF-α or IL-1 production was calculated by the equation:

$$\% \text{ inhibition} = [1- (TS-NC)/(PC-NC)] * 100.$$

The $IC_{50}$ value was defined as the substance concentration, at which 50% of TNF-α production were inhibited.

Compounds showing $IC_{50}$ with 20 μM or lower concentrations are active.

**Determination of TNF-α and IL-1 secretion in mouse peritoneal macrophages *in vitro***

In order to obtain peritoneal macrophages, Balb/C mouse strain males, age 8 to 12 weeks, were injected i.p. with 300 μg of zymosan (SIGMA) dissolved in a phosphate buffer (PBS) in a total volume of 0.1 ml/mouse. After 24 hours the mice were euthanized according to the Laboratory Animal Welfare Act. The peritoneal cavity was washed with a sterile physiological solution (5 ml). The obtained peritoneal macrophages were washed twice with a sterile physiological solution and, after the last centrifugation (350 g/10 min), resuspended in RPMI 1640, into which 10% of FBS were added. In order to determine TNF-α secretion, 5x$10^4$ cells/well were cultivated in a total volume of 200 μl for 18 to 24 hours on microtitre plates with a flat bottom (96 wells, Falcon) in RPMI 1640 medium, into which 10% FBS (Fetal Bovine Serum, Biowhittaker) inactivated by heat, 100 units/ml of penicillin, 100 mg/ml of streptomycin, 20 mM HEPES and 50 μM 2-mercaptoethanol (all of GIBCO) were added. The cells were incubated at 37°C in an atmosphere with 5% $CO_2$ and 90% humidity. In a negative control the cells were cultivated only in a medium (NC), whereas in a positive control the TNF-α secretion was triggered by adding 10 ng/ml of lipopolysaccharides (LPS, *E. coli* serotype 0111:B4, SIGMA) (PC). The effect of the substances upon the TNF-α secretion was investigated after adding them into cultures of cells stimulated with LPS (TS). The TNF-α level in the cell supernatant was determined by ELISA procedure (R&D Systems, Biosource). IL-1 level was determined in an assay, identical to an assay for TNF-α by ELISA procedure (R&D Systems). The percentage of inhibition of TNF-α or IL-1 production was calculated by the equation:

$$\% \text{ inhibition} = [1- (TS-NC)/(PC-NC)] * 100.$$

The $IC_{50}$ value was defined as the substance concentration, at which 50% of TNF-α production were inhibited.

Compounds showing $IC_{50}$ with 10 μM or lower concentrations are active.

***In vivo* model of LPS-induced excessive TNF-α or IL-1 secretion in mice**

TNF-α or IL-1 secretion in mice was induced according to the already disclosed method (Badger AM et al., *J. Pharmac. Env. Therap.,* **1996**, *279*:1453-1461). Balb/C males, age 8 to 12 weeks, in groups of 6 to 10 animals were used. The animals were treated p.o. either with a solvent only (in negative and in positive controls) or with solutions of substances 30 minutes prior to i.p. treatment with LPS (*E. coli* serotype 0111:B4, Sigma) in a dosis of 25 μg/animal. Two hours later the animals were euthanized by means of an i.p. Roumpun (Bayer) and Ketanest (Parke-Davis) injection. A blood sample of each animal was taken into a Vacutainer tube (Becton Dickinson) and the plasma was separated according to the producer's instructions. The TNF-α level in the plasma was determined by ELISA procedure (Biosource, R&D Systems) according to the producer's instructions. The test sensitivity was <3pg/ml TNF-α. The EL-1 level was determined by ELISA procedure (R&D Systems). The percentage of inhibition of TNF-α or IL-1 production was calculated by the equation:

$$\% \text{ inhibition} = [1 - (TS\text{-}NC)/(PC\text{-}NC)] * 100.$$

Active are the compounds showing a 30% or more inhibition of TNF-α production at a dosis of 10 mg/kg.

**Writhing assay for analgetic activity**

In this assay pain is induced by the injection of an irritant, most frequently acetic acid, into the peritoneal cavity of mice. Animals react with characteristic writhings, which has given the name to the assay (Collier HOJ et al., Pharmac. Chemother., 1968, 32:295-310; Fukawa K et al., J. Pharmacol. Meth., 1980, 4:251-259; Schweizer A et al., Agents Actions, 1988, 23:29-31). The assay is convenient for the determination of analgetic activity of compounds. Procedure: male Balb/C mice (Charles River, Italy), age 8 to 12 weeks, were used. A control group received methyl cellulose p.o. 30 minutes prior to i.p. application of acetic acid in a concentration of 0.6%, whereas test groups received standard (acetylsalicylic acid) or test substances in methyl cellulose p.o. 30 minutes prior to i.p. application of 0.6% acetic acid (volume 0.1 ml/10 g). The mice were placed individually under glass funnels and the number of writhings was registered for 20 minutes for each animal. The percentage of writhing inhibition was calculated according to the equation:

**% inhibition = (mean value of number of writhings in the control group – number of writhings in the test group)/number of writhings in the control group * 100.**

Active are the compounds showing such analgetic activity as acetylsalicylic acid or better.

***In vivo* model of LPS-induced shock in mice**

Male Balb/C mice (Charles River, Italy), age 8 to 12 weeks, were used. LPS isolated from *Serratia marcescens* (Sigma, L-6136) was diluted in sterile physiological solution. The first LPS injection was administered intradermally in a dosis of 4 μg/mouse. 18 to 24 hours later, LPS was administered i.v. in a dosis of 200 μg/mouse. A control group received two LPS injections as disclosed above. The test groups received substances p.o. half an hour prior to each LPS application. The survival after 24 hours was observed.

Active are the substances at which the survival at a dosis of 30 mg/kg was 40% or more.

Compounds from Examples 4 and 14 show activity in at least two investigated assays though these results only represent an illustration of the biological activity of the compounds.

**PREPARATION PROCESSES WITH EXAMPLES**

The present invention is illustrated by the following Examples.

**Example 1**

*10-Benzyloxy-8-oxa-1-thia-dibenzo*[e,h]*azulene-2-carboxylic acid ethyl ester*

To a solution of *10-hydroxy-8-oxa-1-thia-dibenzo*[e,h]*azulene-2-carboxylic acid ethyl ester* (**3**) (0.30 mmol) in *N,N*-dimethylformamide (2.0 mL), potassium carbonate (0.74 mmol) was added. The reaction mixture was heated to 100°C, benzyl chloride (0.36 mmol) was added and then the heating of the reaction mixture at 100°C was continued for another 2 hours. After 2 hours the reaction mixture was cooled to room temperature and filtered. The filtrate was evaporated under reduced pressure. Water was added to the evaporation residue and it was extracted with ethyl acetate, dried over anhydrous $Na_2SO_4$ and evaporated under reduced pressure. After purifying by chromatography over a silicagel column a crystalline product was isolated.
$^1$H NMR (ppm, $CDCl_3$): 8.01 (s, 1H), 7.52-6.82 (m, 12H), 5.09 (s, 2H), 4.39 (q, 2H), 1.42 (t, 3H);
MS (*m*/*z*): 429.4 [$MH^+$].

**Example 2**

*10-(3-Dimethylamino-propoxy)-8-oxa-1-thia-dibenzo*[e,h]*azulene-2-carboxylic acid methyl ester*

According to the process disclosed in Example 1, from *10-hydroxy-8-oxa-1-thia-dibenzo*[e,h]*azulene-2-carboxylic acid ethyl ester* (**3**) (0.30 mmol) and 3-dimethylaminopropylchloride hydrochloride (0.36 mmol) an oily product was obtained.
$^1$H NMR (ppm, $CDCl_3$): 8.01 (s, 1H), 7.51-6.74 (m, 7H), 4.39 (q, 2H), 4.07 (t, 2H), 2.59 (t, 2H), 2.37 (s, 6H), 2.06 (m, 2H), 1.42 (t, 3H);
MS (*m*/*z*): 424.3 [$MH^+$].

**Example 3**

*10-(2-Dimethylamino-ethoxy)-8-oxa-1-thia-dibenzo*[e,h]*azulene-2-carboxylic acid ethyl ester*

According to the process disclosed in Example 1, from *10-hydroxy-8-oxa-1-thia-dibenzo*[e,h]*azulene-2-carboxylic acid ethyl ester* (**3**) (0.30 mmol) and 2-dimethylaminoethylchloride hydrochloride (0.36 mmol) an oily product was obtained. $^1$H NMR (ppm, $CDCl_3$): 8.07 (s, 1H), 7.51-6.77 (m, 7H), 4.39 (q, 2H), 4.19 (t, 2H), 2.88 (t, 2H), 2.47 (s, 6H), 1.42 (t, 3H);
MS (*m*/*z*): 410.3 [$MH^+$].

**Example 4**

*10-(2-Pyrrolidin-1-yl-ethoxy)-8-oxa-1-thia-dibenzo*[e,h]*azulene-2-carboxylic acid ethyl ester*

According to the process disclosed in Example 1, from *10-hydroxy-8-oxa-1-thia-dibenazo*[e,h]*azulene-2-carboxylic acid ethyl ester* (**3**) (0.30 mmol) and 1-(2-chloroethyl)-pyrrolidine hydrochloride (0.36 mmol) an oily product was obtained.
$^1$H NMR (ppm, $CDCl_3$): 7.97 (s, 1H), 7.47-6.73 (m, 7H), 4.35 (q, 2H), 4.16 (t, 2H), 2.95 (t, 2H), 2.69 (bs, 4H), 1.37 (t, 3H), 1.82 (bs, 4H);
MS *(m/z):* 436.3 [$MH^+$].

**Example 5**

*11-Benzyloxy-8-oxa-1-thia-dibenzo*[e,h]*azulene-2-carboxylic acid ethyl ester*

According to the process disclosed in Example 1, from *11-hydroxy-8-oxa-1-thia-dibenzo*[e,h]*azulene-2-carboxylic acid ethyl ester* (**4**) (0.59 mmol) and benzyl chloride (0.71 mmol) an oily product was obtained.
$^1$H NMR (ppm, $CDCl_3$): 8.04 (s, 1H), 7.54-6, 98 (m, 12H), 5.08 (s, 2H), 4.43 (m, 2H), 1.45 (t, 3H).

**Example 6**

*11-(3-Dimethylamino-propoxy)-8-oxa-1-thia-dibenzo*[e,h]*azulene-2-carboxylic acid ethyl ester*

According to the process disclosed in Example 1, from *11-hydroxy-8-oxa-1-thia-dibenzo*[e,h]*azulene-2-carboxylic acid ethyl ester* (**4**) (0.74 mmol) and 3-dimethylaminopropylchloride hydrochloride (0.89 mmol) an oily product was obtained.
$^1$H NMR (ppm, $CDCl_3$): 8.02 (s, 1H), 7.52-6.89 (m, 7H), 4.41 (q, 2H), 4.02 (t, 2H), 2.58 (t, 2H), 2.36 (s, 6H), 2.03 (m, 2H), 1.42 (t, 3H);
MS *(m/z):* 424.3 [$MH^+$].

**Example 7**

*10-(3-Dimethylamino-propoxy)-8-oxa-1-thia-dibenzo*[e,h]*azulene-2-carboxylic acid*

To a solution of *10-(3-dimethylamino-propoxy)-8-oxa-1-thia-dibenzo*[e,h]*azulen&e-2-carboxylic acid ethyl ester-* (0.10 mmol) in ethanol (2.0 mL) a 1M sodium hydroxide solution (1.0 mL) was added. The reaction mixture was stirred at 80 °C for 2 hours, then it was cooled to room temperature, diluted with water and extracted with ethyl acetate. The aqueous extract was acidified with concentrated hydrochloric acid to pH 5-6 and a white crystalline product was precipitated and filtered off.
MS *(mlz):* 396.2 [$MH^+$].

**Example 8**

*(10-Benzyloxy-8-oxa-1-thia-dibenzo*[e,h]*azulence-2-yl)-methanol*

To a suspension of lithium aluminum hydride (0.92 mmol) in dry diethyl ether (5.0 mL) a solution of *10-benzyloxy-8-oxa-1-thia-dibenzo*[e,h]*azulene-2-carboxylic acid ethyl ester* (0.46 mmol) in dry diethyl ether (5.0 mL) was added drop by drop. The reaction mixture was stirred for 2 hours at room temperature, then the excess of lithium aluminum hydride was decomposed by adding diethyl ether and water. The obtained white precipitate was filtered off and the filtrate was evaporated under reduced pressure after drying over anhydrous $Na_2SO_4$. After purifying the evaporation residue by chromatography on a silica gel column, a crystalline product was isolated.
$^1$H NMR (ppm, $CDCl_3$): 7.45-6.79 (m, 13H), 5.08 (s, 2H), 4.89 (s, 2H);
MS (*mlz*)*:* 409.2 [$MNa^+$].

**Example 9**

*(11-Benzyloxy-8-oxa-1-thia-dibenzo*[e,h]*azulene-2-yl)-methanol*

According to the process disclosed in Example 8, from *11-benzyloxy-8-oxa-1-thia-dibenzo*[e,h]*azulene-2-carboxylic acid ethyl ester* (0.41 mmol) a crystalline product was obtained.
$^1$H NMR (ppm, $CDCl_3$): 7.46-6.89 (m, 13H), 5.05 (s, 2H), 4.89 (s, 2H);
MS (*m*/*z*): 384.8[$M^-$].

**Example 10**

*[10-(3-Dimethylamino-propoxy)-8-oxa-1-thia-dibenzo*[e,h]*azulence-2-yl]-methanol*

According to the process disclosed in Example 8, from *10-(3-dimethylamino-propoxy)-8-oxa-1-thia-dibenzo* [e,h]*azulene-2-carboxylic acid ethyl ester* (0.65 mmol) an oily product was obtained.
$^1$H NMR (ppm, $CDCl_3$): 7.45-6.67 (m, 8H), 4.88 (s, 2H), 4.01 (t, 2H), 2.58 (t, 2H), 2.36 (s, 6H), 2.03 (m, 2H);
MS *(m/z):* 382.4 [$MH^+$].

**Example 11**

*[11-(3-Dimethylamino-propoxy)-8-oxa-1-thia-dibenzo*[e,h]*azulene-2-yl]-methanol*

According to the process disclosed in Example 8, from *11-(3 -dimethylamino-propoxy)-8-oxa-1-thia-dibenzo*

[e,h]*azulene-2-carboxylic acid ethyl ester* (0.44 mmol) an oily product was obtained.
$^1$H NMR (ppm, $CDCl_3$): 7.46-6.76 (m, 8H), 4.89 (s, 2H), 3.97 (t, 2H), 2.55 (t, 2H), 2.34 (s, 6H), 1.99 (m, 2H);
MS *(m/z):* 382.1 [$MH^+$].

**Example 12**

*[3-(10-Benzyloxy-8-oxa-1-thia-dibenzo*[e,h]*azulene-2-ylmethoxy)-propyl]-dimethylamine*

To a solution of 3-dimethylaminopropylchloride hydrochloride (3.34 mmol) in 50% sodium hydroxide (3.0 mL), a catalytic amount of benzyltriethylammonium chloride and a solution of *(10-benzyloxy-8-oxa-1-thia-dibenzo*[e,h]*azulene-2-yl)-methanol* (0.24 mmol) in toluene (7.7 mL) were added. The reaction mixture was heated for 3 hours under vigorous stirring and reflux, then cooled to room temperature, diluted with water and extracted with dichloromethane. The organic extract was washed with water, dried over anhydrous $Na_2SO_4$ and evaporated under reduced pressure. After purifying by chromatography on a silica gel column an oily product was isolated.
$^1$H NMR (ppm, $CDCl_3$): 7.46-6.79 (m, 13H), 5.07 (s, 2H), 4.69 (s, 2H), 3.60 (t, 2H), 2.42 (t, 2H), 2.26 (s, 6H), 1.83 (m, 2H);
MS (*m/z*): 472.3 [$MH^+$].

**Example 13**

*[3-(11-Benzyloxy-8-oxa-1-thia-dibenzo*[e,h]*azulene-2-ylmethoxy)-propyl]-dimethylamine*

According to the process disclosed in Example 12, from *(11-benzyloxy-8-oxa-1-thia-dibenzo*[e,h] *azulene-2-yl)-methanol* (0.26 mmol) and 3-dimethylaminopropylchloride hydrochloride (3.63 mmol) an oily product was obtained.
$^1$H NMR (ppm, $CDCl_3$): 7.47-6.89 (m, 13H), 5.05 (s, 2H), 4.72 (s, 2H), 3.64 (t, 2H), 2.59 (t, 2H), 2.39 (s, 6H), 1.93 (m, 2H);
MS (*m/z*): 472.2 [$MH^+$].

**Example 14**

*{3-[2-(3-Dimethylamino-propoxymethyl)-8-oxa-1-thia-dibenzo*[e,h]*azulene-10-yloxy]-propyl}-dimethyl-amine*

According to the process disclosed in Example 12, from *[10-(3-dimethylamino-propoxy)-8-oxa-1-thia-dibenzo* [e,h]*azulene-2-yl]-methanol* (0.34 mmol) and 3-dimethylaminopropylchloride hydrochloride (4.77 mmol) a crystalline product was obtained.
$^1$H NMR (ppm, $CDCl_3$): 7.46-6.72 (m, 8H), 4.70 (s, 2H), 4.04 (t, 2H), 3.62 (t, 2H), 2.51-2.47 (m, 4H), 2.32 (s, 6H), 2.29 (s, 6H), 1.99 (m, 2H), 1.88 (m, 2H);
MS *(mlz):* 467.4 [$MH^+$].

**Example 15**

*{3-[2-(3-Dimethylamino-propoxymethyl)-8-oxa-1-thia-dibenzo*[e,h]*azulene-11-yloxy]-propyl}-dimethyl-amine*

According to the process disclosed in Example 12, from *[11-(3-dimethylamino-propoxy)-8-oxa-1-thia-dibenzo* [e,h]*azulene-2-yl]-methanol* (0.39 mmol) and 3-dimethylaminopropylchloride hydrochloride (5.52 mmol) an oily product was obtained.
MS *(m/z):* 467.4 [$MH^+$].

**Example 16** (Comparative Example)

*10-Methoxy-8-oxa-1-thia-dibenzo*[e,h]*azulene-2-carboxylic acid 2-dimethylamino-ethyl ester*

To a solution of *10-methoxy-8-oxa-1-thia-dibenzo*[e,h]*azulene-2-carboxylic acid* (5) (0.31 mmol) in *N,N*-dimethylformamide (2.0 mL), potassium carbonate (0.75 mmol) was added. The reaction mixture was heated to 100°C, 2-dimethylaminoethylchloride hydrochloride (0.37 mmol) was added and then the heating of the reaction mixture at 100°C was continued for another 2 hours. After 2 hours the reaction mixture was cooled to room temperature and filtered. The filtrate was evaporated under reduced pressure. To the evaporation residue water was added, it was extracted with ethyl acetate, dried over anhydrous $Na_2SO_4$ and evaporated under reduced pressure. After purifying by chromatography on a silica gel column an oily product was isolated.
$^1$H NMR (ppm, $CDCl_3$): 8.04 (s, 1H), 7.51-6.77 (m, 7M, 4.52 (t, 2H), 3.85 (s, 3H), 2.86 (bs, 2H), 2.46 (s, 6H);

MS (*m*/*z*): 396.4 [MH+]

**Example 17** (Comparative Example)

*10-Methoxy-8-oxa-1-thia-dibenzo*[e,h]*azulene-2-carboxylic acid 3-dimethylamino-propyl ester*

According to the process disclosed in Example 16, from *10-methoxy-8-oxa-1-thia-dibenzo*[e,h]*azulene-2-carboxylic acid* (5) (0.31 mmol) and 3-dimethylaminopropylchloride hydrochloride (0.37 mmol) an oily product was obtained.
$^1$H NMR (ppm, $CDCl_3$): 8.01 (s, 1H), 7.51-6.77 (m, 7H), 4.42 (t, 2H), 3.85 (s, 3H), 2.66 (bs, 2w, 2.45 (s, 6H), 2.10 (bs, 2H);
MS (*m*/*z*): 410.4 [MH+].

**Example 18** (Comparative Example)

*11-Chloro-8-oxa-1-thia-dibenzo*[e,h]*azulene-2-carboxylic acid 2-dimethylamino-ethyl ester*

According to the process disclosed in Example 16, from *11-chloro-8-oxa-1-thia-dibenzo*[e,h]*azulene-2-carboxylic acid* (0.31 mmol) and 2-dimethylaminoethylchloride hydrochloride (0.37 mmol) a crystalline product was obtained.
$^1$H NMR (ppm, $CDCl_3$): 8.06 (s, 1H), 7.53-7.22 (m, 7H), 4.52 (t, 2H), 2.84 (t, 2H), 2.44 (s, 6H);
MS (*m*/*z*): 400.3 [MH+].

**Examples 19** (Comparative Example)

*11-Chloro-8-oxa-1-thia-dibenzo*[e,h]*azulene-2-carboxylic acid 3-dimethylamino-propyl ester*

According to the process disclosed in Example 16, from *11-chloro-8-oxa-1-thia-dibenzo*[e,h]*azulene-2-carboxylic acid* (0.31 mmol) and 3-dimethylaminopropylchloride hydrochloride (0.37 mmol) a crystalline product was obtained.
$^1$H NMR (ppm, $CDCl_3$): 8.03 (s, 1H), 7.53-7.19 (m, 7H), 4.42 (t, 2H), 2.55 (t, 2H), 2.36 (s, 6H), 2.03 (m, 2H);
MS (*m*/*z*): 414.3 [MH+].

**Example 20**

*10-Hydroxy-8-oxa-1-thia-dibenzo*[e,h]*azulene-2-carboxylic acid 3-dimethylamino-propylester* (Comparative Example)

*10-(3-dimethylamino-propoxy)-8-oxa-1-thia-dibenzo*[e,h]*azulene-2-carboxylic acid 3-dimethylamino-propyl ester*

According to the process disclosed in Example 16, from *10-hydroxy-8-oxa-1-thia-dibenzo*[e,h]*azulene-2-carboxylic acid* (7) (0.19 mmol) and 3-dimethylaminopropylchloride hydrochloride (0.23 mmol) there were obtained 10-*hydroxy-8-oxa-1-thia-dibenzo* [e,h]*azulene-2-carboxylic acid 3-dimethylamino-propyl ester;*
MS *(mlz):* 396.0 [MH+];
and *10-(3-dimethylamino-propoxy)-8-oxa-1-thia-dibenzo*[e,h]*azulene-2-carboxylic acid 3-dimethylamino-propyl ester;*
MS *(mlz):* 481.4 [MH+].

**Example 21**

*Dimethyl-[3-(8-oxa-1-thia-dibenzo*[e,h]*azulene-10-yloxy) propyl]-amine*

To a solution of *8-oxa-1-thia-dibenzo*[e,h]*azulene-10-ol* (**11**) (0.11 mmol) in *N,N*-dimethylformamide (0.7 mL), potassium carbonate (0.28 mmol) was added. The reaction mixture was heated to 100°C, 3-dimethylaminopropylchloride hydrochloride (0.14 mmol) was added and then the heating of the reaction mixture at 100°C was continued for another 2 hours. After 2 hours the reaction mixture was cooled to room temperature and filtered. The filtrate was evaporated under reduced pressure. To the evaporation residue water was added and it was extracted with ethyl acetate, dried over anhydrous $Na_2SO_4$ and evaporated under reduced pressure. After purifying by chromatography on a silica gel column an oily product was isolated.
MS (*m*/*z*): 352.3 [MH+].

**PREPARATION OF STARTING COMPOUNDS AND INTERMEDIATES**

**Method A**

*10-Hydroxy-8-oxa-1-thia-dibenzo*[e,h]*azulene-2-carboxylic acid ethyl ester (3)*

To a solution of *10-methoxy-8-oxa-1-thia-dibenzo*[e,h]*azulene-2-carboxylic acid ethyl ester* (1) (0.43 mmol) in dichloromethane (3.40 mL), a 0.1 M solution of boron(III) bromide in dichloromethane (2.56 mL) was added under stirring at 0°C. The reaction mixture was stirred for 4 hours at room temperature, then water was added and it was extracted with dichloromethane. The organic extract was dried over anhydrous $Na_2SO_4$ and evaporated under reduced pressure. After purifying by chromatography on a silica gel column a crystalline product was isolated.

By an analogous process starting from *11-methoxy-8-oxa-1-thia-dibenzo*[e,h]*azulene-2-carboxylic acid ethyl ester* (**2**)*, 11-hydroxy-8-oxa-1-thia-dibenzo*[e,h]*azulene-2-carboxylic acid ethyl ester* (**4**) was prepared.

**Method B**

*10-Methoxy-8-oxa-1-thia-dibenzo*[e,h]*azulene-2-carboxylic acid* (**5**)

To a solution of *10-methoxy-8-oxa-1-thia-dibenzo*[e,h]*azulene-2-carboxylic acid ethyl ester* (1) (1.37 mmol) in ethanol (10.0 mL), a 1M sodium hydroxide solution (11.2 mL) was added. The reaction mixture was stirred at 80°C for 2 hours, then it was cooled to room temperature, diluted with water and extracted with ethyl acetate. The aqueous extract was acidified with concentrated hydrochloric acid to pH 5-6 and a white crystalline product was precipitated, which was filtered off.

By an analogous process starting from:

*11-mthoxy-8-oxa-1-thia-dibenzo*[e,h]*azulene-2-carboxylic acid ethyl ester* (**2**)*;*
*10-hydroxy-8-oxa-1-thia-dibenzo*[e,h]*azulene-2-carboxylic acid ethyl ester* (**3**)*;*
*11-hydroxy-8-oxa-1-thia-dibenzo*[e,h]*azulene-2-carboxylic acid ethyl ester* (**4**);
there were respectively prepared:
*11-methoxy-8-oxa-1-thia-dibenzo*[e,h]*azulene-2-carboxylic acid* (**6**);
*10-hydroxy-8-oxa-1-thia-dibenzo*[e,h]*azulene-2-carboxylic acid* (**7**);
*11-hydroxy-8-oxa-1-thia-dibenzo*[e,h]*azulene-2-carboxylic acid* (**8**).

**Method C**

*10-Methoxy-8-oxa-1-thia-dibenzo*[e,h]*azulene* (**9**)

A finely ground mixture of *10-methoxy-8-oxa-1-thia-dibenzo*[e,h]*azulene-2-carboxylic acid* (5) (0.31 mmol) and elementary copper (75.0 mg) was heated at 230°C for about 2 hours. The reaction mixture was cooled to room temperature, dissolved in ethyl acetate and washed with water. The organic extract was dried over anhydrous $Na_2SO_4$ and evaporated under reduced pressure. After purifying by chromatography on a silica gel column a crystalline product was isolated.

By an analogous process starting from:
*11-methoxy-8-oxa-1-thia-dibenzo*[e,h]*azulene-2-carboxylic acid* (**6**)*;*
*10-hydroxy-8-oxa-1-thia-dibenzo*[e,h]*azulene-2-carboxylic acid* (**7**)*;*
*11-hydroxy-8-oxa-1-thia-dibenzo*[e,h]*azulene-2-carboxylic acid* (**8**)*;*
there were respectively prepared:
*11-methoxy-8-oxa-1-thia-dibenzo*[e,h]*azulene* (**10**);
*10-hydroxy-8-oxa-1-thia-dibenzo*[e,h]*azulene* (**11**);
*11-hydroxy-8-oxa-1-thia-dibenzo*[e,h]*azulene* (**12**).

**Table 1**

| Characteristics of the prepared starting compounds and intermediates: | | | | | | |
|---|---|---|---|---|---|---|
| **Cpd.** | **X** | **Y** | **Z** | **$R^1$** | **MS (*m/z*)** | **$^1$H NMR (ppm)** |
| **1** | O | H | 10-$OCH_3$ | $CO_2Et$ | 375.2 ($MNa^+$) | 8.08 (s, 1H), 7.51-6.75 (m, 7H), 4.40 (q, 2H), 3.84 (s, 3H), 1.42 (t, 3H) ($CDCl_3$) |
| **2** | O | H | 11-$OCH_3$ | $CO_2Et$ | 353.2 ($MH^+$) | 8.02 (s, 1H), 7.52-6.89 (m, 7H), 4.41 (q, 2H), 3.81 (s, 3H), 1.42 (t, 3H) ($CDCl_3$) |
| **3** | O | H | 10-OH | $CO_2Et$ | 361.2 ($MNa^+$) 361.2($MNa^+$) | 8.01 (s, 1H), 7.60-6.69 (m, 7H), 4,40 (q, 2H), 1.42 (t, 3H) ($CDCl_3$) 4.40 (q, 2H), 1.42 (t, 3H) ($CDCl_3$) |
| **4** | O | H | 11-OH | $CO_2Et$ | 361.0 ($MNa^+$) ($MNa^+$) | 8.02 (s, 1H), 7.51-6.83 (m, 7H), 4,41 (q, 2H), 1.42 (t, 3H) ($CDCl_3$) |
| **5** | O | H | 10-$OCH_3$ | $CO_2H$ | 325.0 ($MH^+$) | 13.00 (bs, 1H), 8.07 (s, 1H), 7.95-6,89 (m, 7H), 3.83 (s, 3H) (DMSO-$d_6$) |
| **6** | O | H | 11-$OCH_3$ | $CO_2H$ | 325.0 ($MH^+$) | - |
| **7** | O | H | 10-OH | $CO_2H$ | 311.0 ($MH^+$) | 12.00 (bs, 1H), 10.27 (s, 1H), 8.04 (s, 1H), 7.68-6.74 (m, 7H) (DMSO-$d_6$) |
| **8** | O | H | 11-OH | $CO_2H$ | 311.0 ($MH^+$) | - |
| **9** | O | H | 10-$OCH_3$ | H | 281.1 ($MH^+$) | 7.53-6.47 (m, 9H), 3.84 (s, 3H) ($CDCl_3$) |
| **10** | O | H | 11-OCH3 | H | 281.1 ($MH^+$) | - |
| **11** | O | H | 10-OH | H | 266.9 ($MH^+$) | - |
| **12** | O | H | 11-OH | H | 266.9 ($MH^+$) | - |

**Claims**

**1.** Compound of the formula I

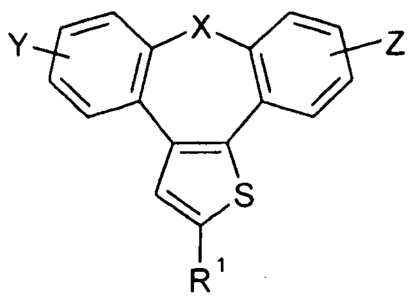

**I**

wherein
X individually denotes a hetero atom -O-;
Y has the meaning of H;

Z has the meaning of a fragment of the formula **II**

$$-O-(CH_2)_m-A \qquad \mathbf{II}$$

wherein

A individually denotes an $C_1$-$C_7$ alkyl which is substituted by 1 to 3 groups selected from halo, hydroxy, $C_1$-$C_4$ alkoxy, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; $C_2$-$C_7$ alkenyl which is unsubstituted or is substituted by 1 to 3 halo atoms; $C_2$-$C_7$ alkinyl; aryl containing one ring with at least 6 carbon atoms or two rings with totally 10 carbon atoms and with alternating double (resonant) bonds between carbon atoms, wherein the aryl group is unsubstituted or substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; $C_4$-$C_{12}$ monocyclic or bicyclic heteroaryl ring system which is aromatic or partially aromatic containing at least one heteroatom selected from oxygen, sulphur and nitrogen, wherein $C_4$-$C_{12}$ monocyclic or bicyclic heteroaryl ring system is unsubstituted or is substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; 5-6 membered heterocyclic group containing at least 1 heteroatom selected from oxygen, sulphur and nitrogen, wherein the 5-6 membered heterocyclic group is fully saturated or partially unsaturated and is unsubstituted or is substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; amino; *N*-($C_1$-$C_7$-alkyl)amino; or *N,N*-di($C_1$-$C_7$-alkyl)amino group;
m denotes an integer from 1 to 4;
$R^1$ individually denotes hydrogen, halo, $C_1$-$C_7$ alkyl which is unsubstituted or is substituted by 1 to 3 groups selected from halo, hydroxy, $C_1$-$C_4$ alkoxy, thiol, $C_1$-$C_4$ alkylthio, amino, *N*-($C_1$-$C_4$) alkylamino, *N,N*-di ($C_1$-$C_4$-alkyl)-amino, sulfonyl, $C_1$-$C_4$ alkylsulfonyl, sulfinyl and $C_1$-$C_4$ alkylsulfinyl; $C_2$-$C_7$ alkenyl which is unsubstituted or is substituted by 1 to 3 halo atoms;, $C_2$-$C_7$ alkinyl; aryl containing one ring with at least 6 carbon atoms or two rings with totally 10 carbon atoms and with alternating double (resonant) bonds between carbon atoms, wherein the aryl group is unsubstituted or is substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; $C_4$-$C_{12}$ monocyclic or bicyclic heteroaryl ring system which is aromatic or partially aromatic containing at least one heteroatom selected from oxygen, sulphur and nitrogen, wherein $C_4$-$C_{12}$ monocyclic or bicyclic heteroaryl ring system is unsubstituted or is substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio, amino, *N*-($C_1$-$C_4$) alkylamino, N,N-di($C_1$-$C_4$-alkyl)-amino, sulfonyl, $C_1$-$C_4$ alkylsulfonyl, sulfinyl and $C_1$-$C_4$ alkylsulfinyl; 5-6 membered heterocyclic group containing at least 1 heteroatom selected from oxygen, sulphur and nitrogen, wherein the 5-6 membered heterocyclic group is fully saturated or partially unsaturated and is unsubstituted or is substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; hydroxy; hydroxy-$C_2$-$C_7$ alkenyl; hydroxy-$C_2$-$C_7$ alkinyl; $C_1$-$C_7$ alkoxy; thiol; thio-$C_2$-$C_7$ alkenyl; thio-$C_2$-$C_7$ alkinyl; $C_1$-$C_7$ alkylthiol; amino; *N*-($C_1$-$C_7$-alkyl)amino; *N,N*-di ($C_1$-$C_7$-alkyl)amino; $C_1$-$C_7$ alkylamino; amino-$C_2$-$C_7$ alkenyl; amino-$C_2$-$C_7$ alkinyl; amino-$C_1$-$C_7$ alkoxy; $C_1$-$C_7$ alkanoyl; aroyl, oxo-$C_1$-$C_7$ alkyl;, $C_1$-$C_7$ alkanoyloxy; carboxy; $C_1$-$C_7$ alkyloxycarbonyl wherein alkyl has the meaning as defined abovearyloxycarbonyl, wherein aryl has the meaning as defined above; carbamoyl;, *N*-($C_1$-$C_7$-alkyl)carbamoyl;, *N,N*-di($C_1$-$C_7$-alkyl)carbamoyl; cyano; cyano-$C_1$-$C_7$ alkyl; sulfonyl; $C_1$-$C_7$ alkylsulfonyl; sulfinyl; $C_1$-$C_7$ alkylsulfinyl; nitro ;or a subtituent of the formula II, wherein the symbols A and m have the meaning as defined above;

and pharmacologically acceptable salts and solvates thereof.

**2.** Compound and salt according to claim 1, wherein the symbol m has the meaning of 1, 2 or 3.

**3.** Compound and salt according to claim 1, wherein A has the meaning of -$N(CH_3)_2$, pyrrolidin-1-yl or phenyl.

**4.** The compound of formula I according to claim 1 selected from the group consisting of:

*10-benzyloxy-8-oxa-1-thia-dibenzo*[e,h]*azulene-2-carboxylic acid ethyl ester;*
*10-(3-dimethylamino-propoxy)-8-oxa-1-thia-dibenzo*[e,h]*azulene-2-carboxylic acid ethyl ester;*
*10-(2-dimethylamino-ethoxy)-8-oxa-1-thia-dibenzo*[e,h]*azulene-2-carboxylic acid ethyl ester;*
*10-(2-pyrrolidin-1-yl-ethoxy)-8-oxa-1-thia-dibenzo*[e,h]*azulene-2-carboxylic acid ethyl ester;*
*11-benzyloxy-8-oxa-1-thia-dibenzo*[e,h]*azulene-2-carboxylic acid methyl ester;*
*11-(3-dimethylamino-propoxy)-8-oxa-1-thia-dibenzo[e,h]azulene-2-carboxylic acid ethyl ester*
*10-(3-dimethylamino-propoxy)-8-oxa-1-thia-dibenzo*[e,h]*azulene-2-carboxylic acid;*
*(10-benzyloxy-8-oxa-1-thia-dibenzo*[*e*,h]*azulene-2-yl)-methanol;*
*(11-benzyloxy-8-oxa-1-thia-dibenzo*[e,h]*azulene-2-yl)-methanol;*
*[10-(3-dimethylamino-propoxy)-8-oxa-1-thia-dibenzo*[e,h]*azulene-2-yl]-methanol;*
*[11-(3-dimethylamino-propoxy)-8-oxa-1-thia-dibenzo*[e,h]*azulene-2-yl]-methanol;*
*[3-(10-benzyloxy-8-oxa-1-thia-dibenzo*[e,h]*azulene-2-ylmethoxy)-propyl]-dimethyl-amine;*
*[3-(11-benzyloxy-8-oxa-1-thia-dibenzo*[e,h]*azulene-2-ylmethoxy)-propyl]-dimethyl-amine;*
*{3-[2-(3-dimethylamino-propoxymethyl)-8-oxa-1-thia-dibenzo*[e,h]*azulene-10-yloxy]-propyl}-dimethyl-amine;*
*{3-[2-(3-dimethylamino-propoxymethyl)-8-oxa-1-thia-dibenzo*[e,h]*azulene-11-yloxy]-propyl}-dimethyl-amine;*
*10-(3-dimethylamino-propoxy)-8-oxa-1-thia-dibenzo*[e,h]*azulene-2-carboxylic acid 3-dimethylamino-propyl ester;* and
*dimethyl-[3-(8-oxa-1-thia-dibenzo*[e,h]*azulene-10-yloxy)-propyl]-amine.*

**5.** The compound of formula I according to claim 4 which is:

*10-(3-dimethylamino-propoxy)-8-oxa-1-thia-dibenzo*[e,h]*azulene-2-carboxylic acid ethyl ester.*

**6.** A process for the preparation of a compound of the formula I

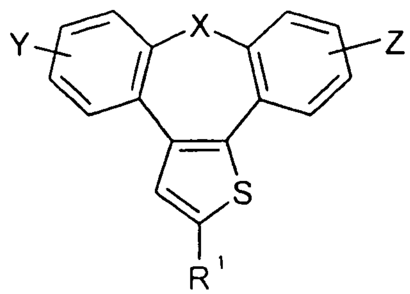

**I**

wherein
X individually denotes a hetero atom -O-;
Y has the meaning of H;
Z has the meaning of a fragment of the formula II

$$-O-(CH_2)_m-A \quad \textbf{II}$$

wherein

A individually denotes an $C_1$-$C_7$ alkyl which is substituted by 1 to 3 groups selected from halo, hydroxy, $C_1$-$C_4$ alkoxy, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; $C_2$-$C_4$ alkenyl which is unsubstituted or is substituted by 1 to 3 halo atoms; $C_2$-$C_7$ alkinyl; aryl containing one ring with at least 6 carbon atoms or two rings with totally 10 carbon atoms and with alternating double (resonant) bonds between carbon atoms, wherein the aryl group is unsubstituted or is substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio, amino, *N*-($C_1$-$C_4$) alkylamino, *N,N*-di ($C_1$-$C_4$-alkyl)-amino, sulfonyl, $C_1$-$C_4$ alkylsulfonyl, sulfinyl and $C_1$-$C_4$ alkylsulfinyl; $C_4$-$C_{12}$ monocyclic or bicyclic heteroaryl ring system which is aromatic or partially aromatic containing at least one heteroatom selected from oxygen, sulphur and nitrogen, wherein $C_4$-$C_{12}$ monocyclic or bicyclic heteroaryl ring system is unsubstituted or

is substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; 5-6 membered heterocyclic group containing at least 1 heteroatom selected from oxygen, sulphur and nitrogen, wherein the 5-6 membered heterocyclic group is fully saturated or partially unsaturated and is unsubstituted or is substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; amino; *N*-($C_1$-$C_7$-alkyl)amino; or *N,N*-di($C_1$-$C_7$-alkyl)amino group;
m denotes an integer from 1 to 4;
$R^1$ individually denotes hydrogen, halo, $C_1$-$C_7$ alkyl which is unsubstituted or is substituted by 1 to 3 groups selected from halo, hydroxy, $C_1$-$C_4$ alkoxy, thiol, $C_1$-$C_4$ alkylthio, amino, *N*-($C_1$-$C_4$) alkylamino, *N,N*-di ($C_1$-$C_4$-alkyl)-amino, sulfonyl, $C_1$-$C_4$ alkylsulfonyl, sulfinyl and $C_1$-$C_4$ alkylsulfinyl; $C_2$-$C_7$ alkenyl which is unsubstituted or is substituted by 1 to 3 halo atoms; $C_2$-$C_7$ alkinyl; aryl containing one ring with at least 6 carbon atoms or two rings with totally 10 carbon atoms and with alternating double (resonant) bonds between carbon atoms, wherein the aryl group is unsubstituted or is substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio; amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; $C_4$-$C_{12}$ monocyclic or bicyclic heteroaryl ring system which is aromatic or partially aromatic containing at least one heteroatom selected from oxygen, sulphur and nitrogen, wherein $C_4$-$C_{12}$ monocyclic or bicyclic heteroaryl ring system is unsubstituted or is substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; 5-6 membered heterocyclic group containing at least 1 heteroatom selected from oxygen, sulphur and nitrogen, wherein the 5-6 membered heterocyclic group is fully saturated or partially unsaturated and is unsubstituted or is substituted by 1 or 2 substituents selected from halo, $C_1$-$C_4$ alkyl, trifluoromethyl, cyano, nitro, hydroxy, $C_1$-$C_4$ alkoxy, $C_1$-$C_4$ alkyloxycarbonyl, thiol, $C_1$-$C_4$ alkylthio, amino, *N*-($C_1$-$C_4$) alkylamino, *N,N*-di($C_1$-$C_4$-alkyl)-amino, sulfonyl, $C_1$-$C_4$ alkylsulfonyl, sulfinyl and $C_1$-$C_4$ alkylsulfinyl; hydroxy; hydroxy-$C_2$-$C_7$ alkenyl; hydroxy-$C_2$-$C_7$ alkinyl; $C_1$-$C_7$ alkoxy; thiol; thio-$C_2$-$C_7$ alkenyl; thio-$C_2$-$C_7$ alkinyl; $C_1$-$C_7$ alkylthiol; amino; *N*-($C_1$-$C_7$-alkyl)amino; *N,N*-di ($C_1$-$C_7$-alkyl)amino; $C_1$-$C_7$ alkylamino; amino-$C_2$-$C_7$ alkenyl; amino-$C_2$-$C_7$ alkinyl; amino-$C_1$-$C_7$ alkoxy; $C_1$-$C_7$ alkanoyl; aroyl; oxo-$C_1$-$C_7$ alkyl; $C_1$-$C_7$ alkanoyloxy; carboxy; $C_1$-$C_7$ alkyloxycarbonyl, wherein alkyl has the meaning as defined above; aryloxycarbonyl, wherein aryl has the meaning as defined above; carbamoyl; *N*-($C_1$-$C_7$-alkyl)carbamoyl; *N,N*-di($C_1$-$C_7$-alkyl)carbamoyl; cyano; cyano-$C_1$-$C_7$ alkyl; sulfonyl; $C_1$-$C_7$ alkylsulfonyl; sulfinyl; $C_1$-$C_7$ alkylsulfinyl; nitro ; or a subtituent of the formula **II**, wherein the symbols A and m have the meaning as defined above;

and pharmacologically acceptable salts and solvates thereof,
**characterized in that** the preparation processes comprises:

a) a condensation reaction of an alcohol of the formula **IIIb**

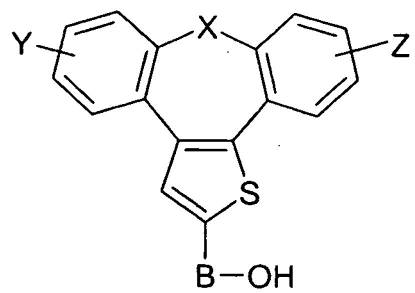

**IIIb**

wherein B individually denotes a -C(O)- or -$CH_2$- group and Y and Z have the meaning as defined above, with a compound of the formula **IVa**

$L^1$ -$(CH_2)_m$ -A **IVa**

wherein $L^1$ has the meaning of a leaving group and m and A have the meaning as defined above; or
b) a condensation reaction of a compound of the formula IIIc

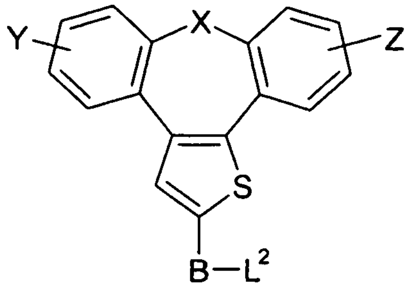

**IIIc**

wherein B individually denotes a -C(O)- or -$CH_2$- group, $L^2$ has the individual meaning of a leaving group and Y and Z have a meaning as defined above,
with a compound of the formula **IVb**

HO-$(CH_2)_m$-A **IVb**

wherein A and m have the meaning as defined above.

**7.** The use of a compound as claimed in any of claims 1 to 5 in the preparation of a medicament for use in the therapy of inflammatory diseases and conditions induced by extensive production of TNF-α and/or IL-1.

**8.** The use according to claim 7, wherein inflammatory diseases and conditions induced by extensive production of TNF-α and/or IL-1 are selected from a group consisting of rheumatoid arthritis, rheumatoid spondylitis, osteoarthritis, eczemas, psoriasis, burns caused by UV radiation, inflammatory eye diseases, Crohn's disease, ulcerative colitis and asthma.

**9.** A pharmaceutical formulation comprising a compound as claimed in any of claims 1 to 5 and pharmaceutically acceptable carrier or solvent.

## Patentansprüche

**1.** Verbindung der Formel I

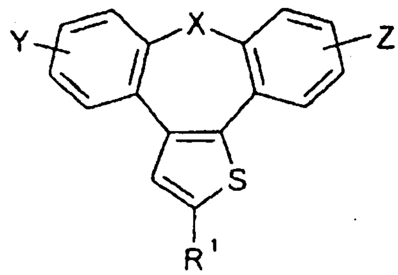

**I**

wobei
X jeweils ein Heteroatom -O- bedeutet;
Y die Bedeutung H hat;
Z die Bedeutung eines Fragments der Formel II hat

$$-O-(CH_2)_m-A \qquad \textbf{II}$$

wobei

A jeweils einen $C_1$-$C_7$-Alkylrest, der mit 1 bis 3 Resten, ausgewählt aus Halogen, Hydroxy, $C_1$-$C_4$-Alkoxy, Thiol, $C_1$-$C_4$-Alkylthio, Amino, N-($C_1$-$C_4$)-Alkylamino, N,N-Di($C_1$-$C_4$-alkyl)-amino, Sulfonyl, $C_1$-$C_4$-Alkylsulfonyl, Sulfinyl und $C_1$-$C_4$-Alkylsulfinyl substituiert ist; einen $C_2$-$C_7$-Alkenylrest, der unsubstituiert oder mit 1 bis 3 Halogenatomen substituiert ist; einen $C_2$-$C_7$-Alkinylrest; einen Arylrest, der einen Ring mit mindestens 6 Kohlenstoffatomen oder zwei Ringe mit insgesamt 10 Kohlenstoffatomen und mit abwechselnden (resonanten) Doppelbindungen zwischen den Kohlenstoffatomen enthält, wobei der Arylrest unsubstituiert oder mit 1 oder 2 Substituenten, ausgewählt aus Halogen, $C_1$-$C_4$-Alkyl, Trifluormethyl, Cyano, Nitro, Hydroxy, $C_1$-$C_4$-Alkoxy, $C_1$-$C_4$-Alkyloxycarbonyl, Thiol, $C_1$-$C_4$-Alkylthio, Amino, N-($C_1$-$C_4$)-Alkylamino, N,N-Di($C_1$-$C_4$-Alkyl)-amino, Sulfonyl, $C_1$-$C_4$-Alkylsulfonyl, Sulfinyl und $C_1$-$C_4$-Alkylsulfinyl, substituiert ist; ein monocyclisches oder bicyclisches $C_4$-$C_{12}$-Heteroarylringsystem, das aromatisch oder teilweise aromatisch ist und mindestens ein Heteroatom, ausgewählt aus Sauerstoff, Schwefel und Stickstoff, enthält, wobei das monocyclische oder bicyclische $C_4$-$C_{12}$-Heteroarylringsystem unsubstituiert oder mit 1 oder 2 Substituenten, ausgewählt aus Halogen, $C_1$-$C_4$-Alkyl, Trifluormethyl, Cyano, Nitro, Hydroxy, $C_1$-$C_4$-Alkoxy, $C_1$-$C_4$-Alkyloxycarbonyl, Thiol, $C_1$-$C_4$-Alkylthio, Amino, N-($C_1$-$C_4$)-Alkylamino,N,N-Di($C_1$-$C_4$-alkyl)-amino, Sulfonyl, $C_1$-$C_4$-Alkylsulfonyl, Sulfinyl und $C_1$-$C_4$-Alkylsulfinyl, substituiert ist; einen 5- bis 6-gliedrigen heterocyclischen Rest, der mindestens ein Heteroatom, ausgewählt aus Sauerstoff, Schwefel und Stickstoff, enthält, wobei der 5- bis 6-gliedrige heterocyclische Rest vollständig gesättigt oder teilweise ungesättigt ist und unsubstituiert oder mit 1 oder 2 Substituenten, ausgewählt aus Halogen, $C_1$-$C_4$-Alkyl, Trifluormethyl, Cyano, Nitro, Hydroxy, $C_1$-$C_4$-Alkoxy, $C_1$-$C_4$-Alkyloxycarbonyl, Thiol, $C_1$-$C_4$-Alkylthio, Amino, N-($C_1$-$C_4$)-Alkylamino, N,N-Di($C_1$-$C_4$-alkyl)-amino, Sulfonyl, $C_1$-$C_4$-Alkylsulfonyl, Sulfinyl und $C_1$-$C_4$-Alkylsulfinyl, substituiert ist; eine Aminogruppe; eine N-($C_1$-$C_7$-Alkyl)aminogruppe; oder eine N, N-Di($C_1$-$C_7$-alkyl)aminogruppe bedeutet;
m eine ganze Zahl von 1 bis 4 bedeutet;
$R^1$ jeweils Wasserstoff, Halogen, $C_1$-$C_7$-Alkyl, das unsubstituiert oder mit 1 bis 3 Resten, ausgewählt aus Halogen, Hydroxy, $C_1$-$C_4$-Alkoxy, Thiol, $C_1$-$C_4$-Alkylthio, Amino, N-($C_1$-$C_4$)-Alkylamino, N,N-Di($C_1$-$C_4$-alkyl)-amino, Sulfonyl, $C_1$-$C_4$-Alkylsulfonyl, Sulfinyl und $C_1$-$C_4$-Alkylsulfinyl, substituiert ist; $C_2$-$C_7$-Alkenyl, das unsubstituiert oder mit 1 bis 3 Halogenatomen substituiert ist; $C_2$-$C_7$-Alkinyl; Aryl, das einen Ring mit mindestens 6 Kohlenstoffatomen oder zwei Ringe mit insgesamt 10 Kohlenstoffatomen und mit abwechselnden (resonanten) Doppelbindungen zwischen den Kohlenstoffatomen enthält, wobei der Arylrest unsubstituiert oder mit 1 oder 2 Substituenten, ausgewählt aus Halogen, $C_1$-$C_4$-Alkyl, Trifluormethyl, Cyano, Nitro, Hydroxy, $C_1$-$C_4$-Alkoxy, $C_1$-$C_4$-Alkyloxycarbonyl, Thiol, $C_1$-$C_4$-Alkylthio, Amino, N-($C_1$-$C_4$)-Alkylamino, N,N-Di($C_1$-$C_4$-alkyl)-amino, Sulfonyl, $C_1$-$C_4$-Alkylsulfonyl, Sulfinyl und $C_1$-$C_4$-Alkylsulfinyl, substituiert ist; ein monocyclisches oder bicyclisches $C_4$-$C_{12}$-Heteroarylringsystem, das aromatisch oder teilweise aromatisch ist und mindestens ein Heteroatom, ausgewählt aus Sauerstoff, Schwefel und Stickstoff, enthält, wobei das monocyclische oder bicyclische $C_4$-$C_{12}$-Heteroarylringsystem unsubstituiert oder mit 1 oder 2 Substituenten, ausgewählt aus Halogen, $C_1$-$C_4$-Alkyl, Trifluormethyl, Cyano, Nitro, Hydroxy, $C_1$-$C_4$-Alkoxy, $C_1$-$C_4$-Alkyloxycarbonyl, Thiol, $C_1$-$C_4$-Alkylthio, Amino, N-($C_1$-$C_4$)-Alkylamino, N,N-Di($C_1$-$C_4$-alkyl)-amino, Sulfonyl, $C_1$-$C_4$-Alkylsulfonyl, Sulfinyl und $C_1$-$C_4$-Alkylsulfinyl, substituiert ist; einen 5- bis 6-gliedrigen heterocyclischen Rest, der mindestens ein Heteroatom, ausgewählt aus Sauerstoff, Schwefel und Stickstoff, enthält, wobei der 5- bis 6-gliedrige heterocyclische Rest vollständig gesättigt oder teilweise ungesättigt ist und unsubstituiert oder mit 1 oder 2 Substituenten, ausgewählt aus Halogen, $C_1$-$C_4$-Alkyl, Trifluormethyl, Cyano, Nitro, Hydroxy, $C_1$-$C_4$-Alkoxy, $C_1$-$C_4$-Alkyloxycarbonyl, Thiol, $C_1$-$C_4$-Alkylthio, Amino, N-($C_1$-$C_4$)-Alkylamino, N,N-Di($C_1$-$C_4$-alkyl)-amino, Sulfonyl, $C_1$-$C_4$-Alkylsulfonyl, Sulfinyl und $C_1$-$C_4$-Alkylsulfinyl, substituiert ist; Hydroxy; Hydrox$_y$-$C_2$-$C_7$-alkenyl; Hydroxy-$C_2$-$C_7$-alkinyl; $C_1$-$C_7$-Alkoxy; Thiol; Thio-$C_2$-$C_7$-alkenyl; Thio-$C_2$-$C_7$-alkinyl; $C_1$-$C_7$-Alkylthio-, Amino; N-($C_1$-$C_7$-Alkyl)amino; N,N-Di ($C_1$-$C_7$-alkyl)amino; $C_1$-$C_7$-Alkylamino, Amino-$C_2$-$C_7$-alkenyl; Amino-$C_2$-$C_7$-alkinyl; Amino-$C_1$-$C_7$-alkoxy; $C_1$-$C_7$-Alkanoyl; Aroyl; Oxo-$C_1$-$C_7$-alkyl; $C_1$-$C_7$-Alkanoyloxy; Carboxy; $C_1$-$C_7$-Alkyloxycarbonyl, wobei Alkyl die vorstehend definierte Bedeutung hat; Aryloxycarbonyl, wobei Aryl die vorstehend definierte Bedeutung hat; Carbamoyl; N-($C_1$-$C_7$-Alkyl)carbamoyl; N,N-Di($C_1$-$C_7$-alkyl)carbamoyl; Cyano; Cyano-$C_1$-$C_7$-alkyl; Sulfonyl; $C_1$-$C_7$-Alkylsulfonyl; Sulfinyl; $C_1$-$C_7$-Alkylsulfinyl; Nitro; oder einen Substituenten der Formel II bedeutet, wobei die Symbole A und m die vorstehend definierte Bedeutung haben;

und pharmakologisch verträgliche Salze und Solvate davon.

**2.** Verbindung und Salz nach Anspruch 1, wobei das Symbol m die Bedeutung 1, 2 oder 3 hat.

**3.** Verbindung und Salz nach Anspruch 1, wobei A die Bedeutung $-N(CH_3)_2$, Pyrrolidin-1-yl oder Phenyl hat.

**4.** Verbindung der Formel I nach Anspruch 1, ausgewählt aus:

10-Benzyloxy-8-oxa-1-thia-dibenzo[e,h]azulen-2-carbonsäureethylester;
10-(3-Dimethylamino-propoxy)-8-oxa-1-thia-dibenzo[e,h]azulen-2-carbonsäureethylester;
10-(2-Dimethylamino-ethoxy)-8-oxa-1-thia-dibenzo[e,h]azulen-2-carbonsäureethylester;
10-(2-Pyrrolidin-1-yl-ethoxy)-8-oxa-1-thia-dibenzo[e,h]azulen-2-carbonsäureethylester;
11-Benzyloxy-8-oxa-1-thia-dibenzo[e,h]azulen-2-carbonsäureethylester;
11-(3-Dimethylamino-propoxy)-8-oxa-1-thia-dibenzo[e,h]azulen-2-carbonsäureethylester;
10-(3-Dimethylamino-propoxy)-8-oxa-1-thia-dibenzo[e,h]azulen-2-carbonsäure;
(10-Benzyloxy-8-oxa-1-thia-dibenzo[e,h]azulen-2-yl)-methanol;
(11-Benzyloxy-8-oxa-1-thia-dibenzo[e,h]azulen-2-yl)-methanol;
[10-(3-Dimethylamino-propoxy)-8-oxa-1-thia-dibenzo[e,h]azulen-2-yl]-methanol;
[11-(3-Dimethylamino-propoxy)-8-oxa-1-thia-dibenzo[e,h]azulen-2-yl]-methanol;
[3-(10-Benzyloxy-8-oxa-1-thia-dibenzo[e,h]azulen-2-ylmethoxy)-propyl]-dimethyl-amin;
[3-(11-Benzyloxy-8-oxa-1-thia-dibenzo[e,h]azulen-2-ylmethoxy)-propyl]-dimethyl-amin;
{3-[2-(3-Dimethylamino-propoxymethyl)-8-oxa-1-thia-dibenzo[e,h]azulen-10-yloxy]-propyl}-dimethyl-amin;
{3-[2-(3-Dimethylamino-propoxymethyl)-8-oxa-1-thia-dibenzo[e,h]azulen-11-yloxy]-propyl} -dimethyl-amin;
10-(3-Dimethylamino-propoxy)-8-oxa-1-thia-dibenzo[e,h]azulen-2-carbonsäure-3-dimethylamino-propylester; und
Dimethyl-[3-(8-oxa-1-thia-dibenzo[e,h]azulen-10-yloxy)-propyl]-amin.

**5.** Verbindung der Formel I nach Anspruch 4, nämlich:

10-(3-Dimethylamino-propoxy)-8-oxa-1-thia-dibenzo[e,h]azulen-2-carbonsäureethylester.

**6.** Verfahren zur Herstellung einer Verbindung der Formel 1

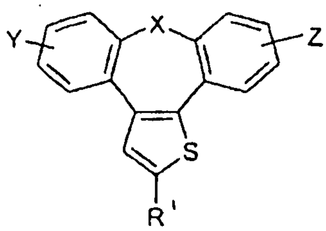

**I**

wobei
X jeweils ein Heteroatom -O- bedeutet;
Y die Bedeutung H hat;
Z die Bedeutung eines Fragments der Formel II hat

$$-O-(CH_2)_m-A \qquad \textbf{II}$$

wobei

A jeweils einen $C_1$-$C_7$-Alkylrest, der mit 1 bis 3 Resten, ausgewählt aus Halogen, Hydroxy, $C_1$-$C_4$-Alkoxy, Thiol, $C_1$-$C_4$-Alkylthio, Amino, N-($C_1$-$C_4$)-Alkylamino, N,N-Di($C_1$-$C_4$-alkyl)-amino, Sulfonyl, $C_1$-$C_4$-Alkylsulfonyl, Sulfinyl und $C_1$-$C_4$-Alkylsulfinyl substituiert ist; einen $C_2$-$C_4$-Alkenylrest, der unsubstituiert oder mit 1 bis 3 Halogenatomen substituiert ist; einen $C_2$-$C_7$-Alkinylrest; einen Arylrest, der einen Ring mit mindestens 6 Kohlenstoffatomen oder zwei Ringe mit insgesamt 10 Kohlenstoffatomen und mit abwechselnden (resonanten) Doppelbindungen zwischen den Kohlenstoffatomen enthält, wobei der Arylrest unsubstituiert oder mit 1 oder 2 Substituenten, ausgewählt aus Halogen, $C_1$-$C_4$-Alkyl, Trifluormethyl, Cyano, Nitro, Hydroxy, $C_1$-$C_4$-Alkoxy, $C_1$-$C_4$-Alkyloxycarbonyl, Thiol, $C_1$-$C_4$-Alkylthio, Amino, N-($C_1$-$C_4$)-Alkylamino, N,N-Di($C_1$-$C_4$-Alkyl)-amino, Sulfonyl, $C_1$-$C_4$-Alkylsulfonyl, Sulfinyl und $C_1$-$C_4$-Alkylsulfinyl, substituiert ist; ein monocyclisches oder bicyclisches $C_4$-$C_{12}$-Heteroarylringsystem, das aromatisch oder teilweise aromatisch ist und mindestens ein Heteroatom, ausgewählt aus Sauerstoff, Schwefel und Stickstoff, enthält, wobei das monocyclische oder bicyclische $C_4$-$C_{12}$-Heteroarylringsystem unsubstituiert oder mit 1 oder 2 Substituenten, ausgewählt aus Halogen, $C_1$-$C_4$-Alkyl, Trifluormethyl, Cyano, Nitro, Hydroxy, $C_1$-$C_4$-Alkoxy, $C_1$-$C_4$-Alkyloxycarbonyl, Thiol, $C_1$-$C_4$-Alkylthio, Amino, N-($C_1$-$C_4$)-Alkylamino, N,N-Di($C_1$-$C_4$-alkyl)-amino, Sulfonyl, $C_1$-$C_4$-Alkylsulfonyl, Sulfinyl und $C_1$-$C_4$-Alkylsulfinyl, substituiert ist; einen 5- bis 6-gliedrigen heterocyclischen Rest, der mindestens ein Heteroatom, ausgewählt aus Sauerstoff, Schwefel und Stickstoff, enthält, wobei der 5- bis 6-gliedrige heterocyclische Rest vollständig gesättigt oder teilweise ungesättigt ist und unsubstituiert oder mit 1 oder 2 Substituenten, ausgewählt aus Halogen, $C_1$-$C_4$-Alkyl, Trifluormethyl, Cyano, Nitro, Hydroxy, $C_1$-$C_4$-Alkoxy, $C_1$-$C_4$-Alkyloxycarbonyl, Thiol, $C_1$-$C_4$-Alkylthio, Amino, N-($C_1$-$C_4$)-Alkylamino, N,N-Di($C_1$-$C_4$-alkyl)-amino, Sulfonyl, $C_1$-$C_4$-Alkylsulfonyl, Sulfinyl und $C_1$-$C_4$-Alkylsulfinyl, substituiert ist; eine Aminogruppe; eine N-($C_1$-$C_7$-Alkyl)aminogruppe; oder eine N, N-Di($C_1$-$C_7$-alkyl)aminogruppe bedeutet;

m eine ganze Zahl von 1 bis 4 bedeutet;

$R^1$ jeweils Wasserstoff, Halogen, $C_1$-$C_7$-Alkyl, das unsubstituiert oder mit 1 bis 3 Resten, ausgewählt aus Halogen, Hydroxy, $C_1$-$C_4$-Alkoxy, Thiol, $C_1$-$C_4$-Alkylthio, Amino, N-($C_1$-$C_4$)-Alkylamino, N,N-Di($C_1$-$C_4$-alkyl)-amino, Sulfonyl, $C_1$-$C_4$-Alkylsulfonyl, Sulfinyl und $C_1$-$C_4$-Alkylsulfinyl, substituiert ist; $C_2$-$C_7$-Alkenyl, das unsubstituiert oder mit 1 bis 3 Halogenatomen substituiert ist; $C_2$-$C_7$-Alkinyl; Aryl, das einen Ring mit mindestens 6 Kohlenstoffatomen oder zwei Ringe mit insgesamt 10 Kohlenstoffatomen und mit abwechselnden (resonanten) Doppelbindungen zwischen den Kohlenstoffatomen enthält, wobei der Arylrest unsubstituiert oder mit 1 oder 2 Substituenten, ausgewählt aus Halogen, $C_1$-$C_4$-Alkyl, Trifluonnethyl, Cyano, Nitro, Hydroxy, $C_1$-$C_4$-Alkoxy, $C_1$-$C_4$-Alkyloxycarbonyl, Thiol, $C_1$-$C_4$-Alkylthio, Amino, N-($C_1$-$C_4$)-Alkylamino, N,N-Di($C_1$-$C_4$-alkyl)-amino, Sulfonyl, $C_1$-$C_4$-Alkylsulfonyl, Sulfinyl und $C_1$-$C_4$-Alkylsulfinyl, substituiert ist; ein monocyclisches oder bicyclisches $C_4$-$C_{12}$-Heteroarylringsystem, das aromatisch oder teilweise aromatisch ist und mindestens ein Heteroatom, ausgewählt aus Sauerstoff, Schwefel und Stickstoff, enthält, wobei das monocyclische oder bicyclische $C_4$-$C_{12}$-Heteroarylringsystem unsubstituiert oder mit 1 oder 2 Substituenten, ausgewählt aus Halogen, $C_1$-$C_4$-Alkyl, Trifluormethyl, Cyano, Nitro, Hydroxy, $C_1$-$C_4$-Alkoxy, $C_1$-$C_4$-Alkyloxycarbonyl, Thiol, $C_1$-$C_4$-Alkylthio, Amino, N-($C_1$-$C_4$)-Alkylamino, N,N-Di($C_1$-$C_4$-alkyl)-amino, Sulfonyl, $C_1$-$C_4$-Alkylsulfonyl, Sulfinyl und $C_1$-$C_4$-Alkylsulfinyl, substituiert ist; einen 5- bis 6-gliedrigen heterocyclischen Rest, der mindestens ein Heteroatom, ausgewählt aus Sauerstoff, Schwefel und Stickstoff, enthält, wobei der 5- bis 6-gliedrige heterocyclische Rest vollständig gesättigt öder teilweise ungesättigt ist und unsubstituiert oder mit 1 oder 2 Substituenten, ausgewählt aus Halogen, $C_1$-$C_4$-Alkyl, Trifluormethyl, Cyano, Nitro, Hydroxy, $C_1$-$C_4$-Alkoxy, $C_1$-$C_4$-Alkyloxycarbonyl, Thiol, $C_1$-$C_4$-Alkylthio, Amino, N-($C_1$-$C_4$)-Alkylamino, N,N-Di($C_1$-$C_4$-alkyl)-amino, Sulfonyl, $C_1$-$C_4$-Alkylsulfonyl, Sulfinyl und $C_1$-$C_4$-Alkylsulfinyl, substituiert ist; Hydroxy; Hydroxy-$C_2$-$C_7$-alkenyl; Hydroxy-$C_2$-$C_7$-alkinyl; $C_1$-$C_7$-Alkoxy; Thiol; Thio-$C_2$-$C_7$-alkenyl; Thio-$C_2$-$C_7$-alkinyl; $C_1$-$C_7$-Alkylthio; Amino; N-($C_1$-$C_7$-Alkyl)amino; N,N-Di ($C_1$-$C_7$-alkyl)amino; $C_1$-$C_7$-Alkylamino, Amino-$C_2$-$C_7$-alkenyl; Amino-$C_2$-$C_7$-alkinyl; Amino-$C_1$-$C_7$-alkoxy; $C_1$-$C_7$-Alkanoyl; Aroyl; Oxo-$C_1$-$C_7$-alkyl; $C_1$-$C_7$-Alkanoyloxy; Carboxy; $C_1$-$C_7$-Alkyloxycarbonyl, wobei Alkyl die vorstehend definierte Bedeutung hat; Aryloxycarbonyl, wobei Aryl die vorstehend definierte Bedeutung hat; Carbamoyl; N-($C_1$-$C_7$-Alkyl)carbamoyl; N,N-Di($C_1$-$C_7$-alkyl)carbamoyl; Cyano; Cyano-$C_1$-$C_7$-alkyl; Sulfonyl; $C_1$-$C_7$-Alkylsulfonyl; Sulfinyl; $C_1$-$C_7$-Alkylsulfinyl; Nitro; oder einen Substituenten der Formel II bedeutet, wobei die Symbole A und m die vorstehend definierte Bedeutung haben;

und pharmakologisch verträgliche Salze und Solvate davon,
**dadurch gekennzeichnet, dass** das Herstellungsverfahren umfasst:

a) eine Kondensationsreaktion eines Alkohols der Formel IIIb

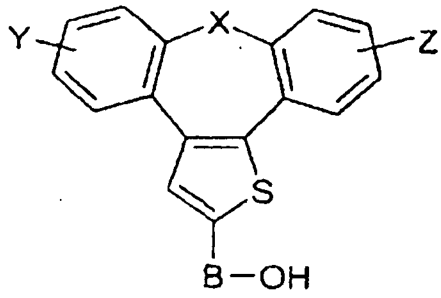

IIIb

wobei B jeweils einen Rest -C(O)- oder $-CH_2-$ bedeutet und Y und Z die vorstehend definierte Bedeutung haben, mit einer Verbindung der Formel IVa

$L^1-(CH_2)_m-A$ **IVa**

wobei $L^1$ die Bedeutung einer Abgangsgruppe hat und m und A die vorstehend definierte Bedeutung haben; oder b) eine Kondensationsreaktion einer Verbindung der Formel IIIc

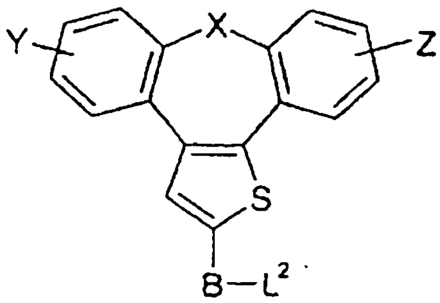

IIIc

wobei B jeweils einen Rest -C(O)- oder $-CH_2-$ bedeutet, $L^2$ die jeweilige Bedeutung einer Abgangsgruppe hat und Y und Z eine vorstehend definierte Bedeutung haben, mit einer Verbindung der Formel IVb

$HO-CH_2)_m-A$ **IVb**

wobei A und m die vorstehend definierte Bedeutung haben.

**7.** Verwendung einer Verbindung nach einem der Ansprüche 1 bis 5 bei der Herstellung eines Medikaments zur Verwendung bei der Therapie entzündlicher Erkrankungen und Zustände, die durch die übermäßige Produktion von TNF-$\alpha$ und/oder IL-1 hervorgerufen werden.

**8.** Verwendung nach Anspruch 7, wobei entzündliche Erkrankungen und Zustände, die durch übermäßige Produktion von TNF-$\alpha$ und/oder IL-1 hervorgerufen werden, aus rheumatoider Arthritis, rheumatoider Spondylitis, Osteoarthritis, Ekzemen, Psoriasis, durch UV-Strahlung verursachten Verbrennungen, entzündlichen Augenerkrankungen, Morbus Crohn, Colitis ulcerosa und Asthma ausgewählt sind.

**9.** Pharmazeutische Formulierung, umfassend eine Verbindung nach einem der Ansprüche 1 bis 5 und einen pharmazeutisch verträglichen Träger oder ein pharmazeutisch verträgliches Lösungsmittel.

## Revendications

**1.** Composé de formule I

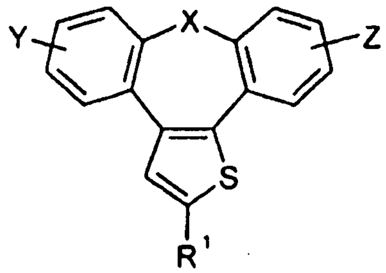

dans laquelle
X représente individuellement un hétéroatome -O- ;
Y représente H ;
Z représente un fragment de formule II

$$-O-(CH_2)_m-A \qquad \textbf{II}$$

dans laquelle

A représente individuellement un groupe alkyle en $C_1$ à $C_7$ qui est substitué avec 1 à 3 groupes choisis entre des groupes halogéno, hydroxy, alkoxy en $C_1$ à $C_4$, thiol, alkylthio en $C_1$ à $C_4$, amino, *N*-(alkyle en $C_1$ à $C_4$)-amino, *N,N*-di(alkyle en $C_1$ à $C_4$)-amino, sulfonyle, alkylsulfonyle en $C_1$ à $C_4$, sulfinyle et alkylsulfinyle en $C_1$ à $C_4$ ; alcényle en $C_2$ à $C_7$ qui est non substitué ou qui est substitué avec 1 à 3 atomes d'halogène ; alcynyle en $C_2$ à $C_7$ ; aryle contenant un noyau ayant au moins 6 atomes de carbone ou bien deux noyaux ayant au total dix atomes de carbone et avec des doubles liaisons alternées (liaisons de résonance) entre les atomes de carbone, le groupe aryle étant non substitué ou substitué avec un ou deux substituants choisis entre des substituants halogéno, alkyle en $C_1$ à $C_4$, trifluorométhyle, cyano, nitro, hydroxy, alkoxy en $C_1$ à $C_4$, (alkyle en $C_1$ à $C_4$) oxycarbonyle, thiol, alkylthio en $C_1$ à $C_4$, amino, *N*-(alkyle en $C_1$ à $C_4$) amino, *N,N*-di-(alkyle en $C_1$ à $C_4$)-amino, sulfonyle, alkylsulfonyle en $C_1$ à $C_4$, sulfinyle et alkylsulfinyle en $C_1$ à $C_4$ ; un système de noyau hétéroaryle en $C_4$ à $C_{12}$ monocyclique ou bicyclique qui est aromatique ou partiellement aromatique, contenant au moins un hétéroatome choisi entre l'oxygène, le soufre et l'azote, ledit système de noyau hétéroaryle en $C_4$ à $C_{12}$ monocyclique ou bicyclique étant non substitué ou étant substitué avec un ou deux substituants choisis entre des substituants halogéno, alkyle en $C_1$ à $C_4$, trifluorométhyle, cyano, nitro, hydroxy, alkoxy en $C_1$ à $C_4$, (alkyle en $C_1$ à $C_4$)-oxycarbonyle, thiol, alkylthio en $C_1$ à $C_4$, amino, *N*-(alkyle en $C_1$ à $C_4$) amino, *N,N*-di(alkyle en $C_1$ à $C_4$)-amino, sulfonyle, alkylsulfonyle en $C_1$ à $C_4$, sulfinyle et alkylsulfinyle en $C_1$ à $C_4$ ; un groupe hétérocyclique penta- ou hexagonal contenant au moins un hétéroatome choisi entre l'oxygène, le soufre et l'azote, ledit groupe hétérocyclique penta- ou hexagonal étant totalement saturé ou partiellement insaturé et étant non substitué ou étant substitué avec un ou deux substituants choisis entre des substituants halogéno, alkyle en $C_1$ à $C_4$, trifluorométhyle, cyano, nitro, hydroxy, alkoxy en $C_1$ à $C_4$, (alkyle en $C_1$ à $C_4$)oxycarbonyle, thiol, alkylthio en $C_1$ à $C_4$, amino, *N*-(alkyle en $C_1$ à $C_4$)-amino, *N,N*-di(alkyle en $C_1$ à $C_4$)-amino, sulfonyle, alkylsulfonyle en $C_1$ à $C_4$, sulfinyle et alkylsulfinyle en $C_1$ à $C_4$ ; amino ; *N*-(alkyle en $C_1$ à $C_7$) amino ; ou *N,N*-di(alkyle en $C_1$ à $C_7$) amino ;
m représente un nombre entier de 1 à 4 ;
$R^1$ représente individuellement un atome d'hydrogène, un groupe halogéno, alkyle en $C_1$ à $C_7$ qui est non substitué ou qui est substitué avec 1 à 3 groupes choisis entre des groupes halogéno, hydroxy, alkoxy en $C_1$ à $C_4$, thiol, alkylthio en $C_1$ à $C_4$, amino, *N*-(alkyle en $C_1$ à $C_4$) amino, *N,N*-di(alkyle en $C_1$ à $C_4$) -amino, sulfonyle, alkylsulfonyle en $C_1$ à $C_4$, sulfinyle et alkylsulfinyle en $C_1$ à $C_4$ ; alcényle en $C_2$ à $C_7$ qui est non substitué ou qui est substitué avec 1 à 3 atomes d'halogène ; alcynyle en $C_2$ à $C_7$ ; aryle contenant un noyau ayant au moins 6 atomes de carbone ou bien deux noyaux ayant au total dix atomes de carbone et avec des doubles liaisons alternées (liaisons de résonance) entre les atomes de carbone, ledit groupe aryle étant non substitué ou étant substitué avec un ou deux substituants choisis entre des substituants halogéno, alkyle en $C_1$ à $C_4$, trifluorométhyle, cyano, nitro, hydroxy, alkoxy en $C_1$ à $C_4$, (alkyle en $C_1$ à $C_4$)-oxycarbonyle, thiol, alkylthio en $C_1$ à $C_4$, amino, *N*-(alkyle en $C_1$ à $C_4$) amino, *N,N*-di(alkyle en $C_1$ à $C_4$)-amino, sulfonyle, alkylsulfonyle en $C_1$ à $C_4$, sulfinyle et alkylsulfinyle en $C_1$ à $C_4$ ; un système de noyau hétéroaryle en $C_4$ à $C_{12}$ monocyclique ou bicyclique

qui est aromatique ou partiellement aromatique, contenant au moins un hétéroatome choisi entre l'oxygène, le soufre et l'azote, ledit système de noyau hétéroaryle en $C_4$ à $C_{12}$ monocyclique ou bicyclique étant non substitué ou étant substitué avec un ou deux substituants choisis entre des substituants halogéno, alkyle en $C_1$ à $C_4$, trifluorométhyle, cyano, nitro, hydroxy, alkoxy en $C_1$ à $C_4$, (alkyle en $C_1$ à $C_4$)oxycarbonyle, thiol, alkylthio en $C_1$ à $C_4$, amino, *N*-(alkyle en $C_1$ à $C_4$)-amino, *N,N*-di(alkyle en $C_1$ à $C_4$)-amino, sulfonyle, alkylsulfonyle en $C_1$ à $C_4$, sulfinyle et alkylsulfinyle en $C_1$ à $C_4$ ; un groupe hétérocyclique penta- ou hexagonal contenant au moins un hétéroatome choisi entre l'oxygène, le soufre et l'azote, ledit groupe hétérocyclique penta- ou hexagonal étant totalement saturé ou partiellement insaturé et étant non substitué ou substitué avec un ou deux substituants choisis entre des substituants halogéno, alkyle en $C_1$ à $C_4$, trifluorométhyle, cyano, nitro, hydroxy, alkoxy en $C_1$ à $C_4$, (alkyle en $C_1$ à $C_4$) oxycarbonyle, thiol, alkylthio en $C_1$ à $C_4$, amino, *N*-(alkyle en $C_1$ à $C_4$)-amino, *N,N*-di-(alkyle en $C_1$ à $C_4$)-amino, sulfonyle, alkylsulfonyle en $C_1$ à $C_4$, sulfinyle et alkylsulfinyle en $C_1$ à $C_4$ ; hydroxy ; hydroxyalcényle en $C_2$ à $C_7$ ; hydroxyalcynyle en $C_2$ à $C_7$ ; alkoxy en $C_1$ à $C_7$ ; thiol ; thioalcényle en $C_2$ à $C_7$ ; thioalcynyle en $C_2$ à $C_7$ ; alkylthiol en $C_1$ à $C_7$ ; amino ; *N*-(alkyle en $C_1$ à $C_7$) amino ; *N,N*-di-(alkyle en $C_1$ à $C_7$)amino ; alkylamino en $C_1$ à $C_7$ ; aminoalcényle en $C_2$ à $C_7$ ; aminoalcynyle en $C_2$ à $C_7$ ; aminoalkoxy en $C_1$ à $C_7$ ; alcanoyle en $C_1$ à $C_7$ ; aroyle ; oxoalkyle en $C_1$ à $C_7$ ; alcanoyloxy en $C_1$ à $C_7$ ; carboxy ; (alkyle en $C_1$ à $C_7$) oxycarbonyle dans lequel le groupe alkyle répond à la définition précitée ; aryloxycarbonyle, dans lequel le groupe aryle répond à la définition précitée ; carbamoyle ; *N*-(alkyle en $C_1$ à $C_7$) carbamoyle ; N,N-di (alkyle en $C_1$ à $C_7$) carbamoyle ; cyano ; cyano-(alkyle en $C_1$ à $C_7$) ; sulfonyle ; alkylsulfonyle en $C_1$ à $C_7$ ; sulfinyle ; alkylsulfinyle en $C_1$ à $C_7$ ; nitro ; ou un substituant de formule II, dans laquelle les symboles A et m répondent aux définitions précitées ;

et ses sels et produits de solvatation pharmacologiquement acceptables.

**2.** Composé et sels suivant la revendication 1, dans lesquels le symbole m est égal à 1, 2 ou 3.

**3.** Composé et sels suivant la revendication 1, dans lesquels A représente un groupe -$N(CH_3)_2$, pyrrolidine-1-yle ou phényle.

**4.** Composé de formule I suivant la revendication 1, choisi dans le groupe consistant en :

*l'ester éthylique d'acide 10-benzyloxy-8-oxa-1-thia-dibenzo*[e,h]*azulène-2-carboxylique ;*
*l'ester éthylique d'acide 10-(3-diméthylamino-propoxy) - 8-oxa-1-thia-dibenzo*[e,h]*azulène-2-carboxylique ;*
*l'ester éthylique d'acide 10-(2-diméthylamino-éthoxy)-8-oxa-1-thia-dibenzo*[e,h]*azulène-2-carboxylique ;*
*l'ester éthylique d'acide 10-(2-pyrrolidine-1-yl-éthoxy) - 8-oxa-1-thia-dibenzo*[e,h]*azulène-2-carboxylique ;*
*l'ester éthylique d'acide 11-benzyloxy-8-oxa-1-thia-dibenzo*[e,h]*azulène-2-carboxylique ;*
*l'ester éthylique d'acide 11-(3-diméthylamino-propoxy)-8-oxa-1-thia-dibenzo*[e,h]*azuléne-2-carboxylique ;*
*l'acide 10-(3-diméthylamino-propoxy)-8-oxa-1-thia-dibenzo-*[e,h]*azulène-2-carboxylique ;*
*le (10-benzyloxy-8-oxa-1-thia-dibenzo*[e,h]*azulène-2-yl)-méthanol ;*
*le (11-benzyloxy-8-oxa-1-thia-dibenzo*[e,h]*azulène-2-yl)-méthanol ;*
*le (10-(3-diméthylamino-propoxy)-8-oxa-1-thia-dibenzo-*[e,h]*azulène-2-yl]méthanol ;*
*le [11-(3-diméthylamino-propoxy)-8-oxa-1-thia-dibenzo-*[e,h]*azulène-2-yl]méthanol ;*
*la [3-(10-benzyloxy-8-oxa-1-thia-dibenzo*[e,h]*azulène-2-ylméthoxy)-propyl]-diméthyl-amine ;*
*la [3-(11-benzyloxy-8-oxa-1-thia-dibenzo*[e,h]*azulène-2-ylméthoxy)-propyl]-diméthyl-amine ;*
*la {3-[2-(3-diméthylamino-propoxyméthyl)-8-oxa-1-thia-dibenzo*[e,h]*azulène-10-yloxy]-propyl}-diméthyl-amine ;*
*la {3-[2-(3-diméthylamino-propoxyméthyl)-8-oxa-1-thia-dibenzo*[e,h]*azulène-11-yloxy]-propyl}-diméthyl-amine ;*
*l'ester 3-diméthylaminopropylique d'acide 10-(3-diméthyl-amino-propoxy)-8-oxa-1-thia-dibenzo*[e,h]*azulène-2-carboxylique ; et*
*la diméthyl-[3-(8-oxa-1-thia-dibenzo*[e,h]*azulène-10-yloxy)-propyl]-amide.*

**5.** Composé de formule I suivant la revendication 4, qui est :

*l'ester éthylique d'acide 10-(3-diméthylamino-propoxy) - 8-oxa-1-thia-dibenzo*[e,h]*azulène-2-carboxylique.*

**6.** Procédé pour la préparation d'un composé de formule I

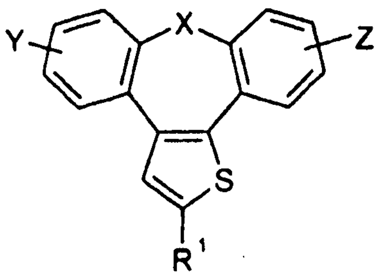

dans laquelle
X représente individuellement un hétéroatome -O- ;
Y représente H ;
Z représente un fragment de formule II

$$-O-(CH_2)_m-A \qquad \textbf{II}$$

dans laquelle

A représente individuellement un groupe alkyle en $C_1$ à $C_7$ qui est substitué avec 1 à 3 groupes choisis entre des groupes halogéno, hydroxy, alkoxy en $C_1$ à $C_4$, thiol, alkylthio en $C_1$ à $C_4$, amino, *N*-(alkyle en $C_1$ à $C_4$)-amino, *N,N*-di(alkyle en $C_1$ à $C_4$)-amino, sulfonyle, alkylsulfonyle en $C_1$ à $C_4$, sulfinyle et alkylsulfinyle en $C_1$ à $C_4$ ; alcényle en $C_2$ à $C_4$ qui est non substitué ou qui est substitué avec 1 à 3 atomes d'halogène, alcynyle en $C_2$ à $C_7$ ; aryle contenant un noyau ayant au moins 6 atomes de carbone ou bien deux noyaux ayant au total dix atomes de carbone et avec des doubles liaisons alternées (liaisons de résonance) entre les atomes de carbone, ledit groupe aryle étant non substitué ou substitué avec un ou deux substituants choisis entre des substituants halogéno, alkyle en $C_1$ à $C_4$, trifluorométhyle, cyano, nitro, hydroxy, alkoxy en $C_1$ à $C_4$, (alkyle en $C_1$ à $C_4$) oxycarbonyle, thiol, alkylthio en $C_1$ à $C_4$, amino, *N*-(alkyle en $C_1$ à $C_4$)amino, *N,N*-di-(alkyle en $C_1$ à $C_4$)-amino, sulfonyle, alkylsulfonyle en $C_1$ à $C_4$, sulfinyle et alkylsulfinyle en $C_1$ à $C_4$ ; un système de noyau hétéroaryle en $C_4$ à $C_{12}$ monocyclique ou bicyclique qui est aromatique ou partiellement aromatique, contenant au moins un hétéroatome choisi entre l'oxygène, le soufre et l'azote, ledit système de noyau hétéroaryle en $C_4$ à $C_{12}$ monocyclique ou bicyclique étant non substitué ou étant substitué avec un ou deux substituants choisis entre des substituants halogéno, alkyle en $C_1$ à $C_4$, trifluorométhyle, cyano, nitro, hydroxy, alkoxy en $C_1$ à $C_4$, (alkyle en $C_1$ à $C_4$)-oxycarbonyle, thiol, alkylthio en $C_1$ à $C_4$, amino, *N*-(alkyle en $C_1$ à $C_4$)amino, *N,N*-di(alkyle en $C_1$ à $C_4$)-amino, sulfonyle, alkylsulfonyle en $C_1$ à $C_4$, sulfinyle et alkylsulfinyle en $C_1$ à $C_4$ ; un groupe hétérocyclique penta- ou hexagonal contenant au moins un hétéroatome choisi entre l'oxygène, le soufre et l'azote, ledit groupe hétérocyclique penta- ou hexagonal étant totalement saturé ou partiellement insaturé et étant non substitué ou étant substitué avec un ou deux substituants choisis entre des substituants halogéno, alkyle en $C_1$ à $C_4$, trifluorométhyle, cyano, nitro, hydroxy, alkoxy en $C_1$ à $C_4$, (alkyle en $C_1$ à $C_4$)oxycarbonyle, thiol, alkylthio en $C_1$ à $C_4$, amino, *N*- (alkyle en $C_1$ à $C_4$)-amino, *N,N*-di(alkyle en $C_1$ à $C_4$)-amino, sulfonyle, alkylsulfonyle en $C_1$ à $C_4$, sulfinyle et alkylsulfinyle en $C_1$ à $C_4$ ; amino ; *N*-(alkyle en $C_1$ à $C_7$) amino ; ou *N,N*-di(alkyle en $C_1$ à $C_7$)amino ;
m représente un nombre entier de 1 à 4 ;
$R^1$ représente individuellement un atome d'hydrogène, un groupe halogéno, alkyle en $C_1$ à $C_7$ qui est non substitué ou qui est substitué avec 1 à 3 groupes choisis entre des groupes halogéno, hydroxy, alkoxy en $C_1$ à $C_4$, thiol, alkylthio en $C_1$ à $C_4$, amino, *N*-(alkyle en $C_1$ à $C_4$) amino, *N,N*-di(alkyle en $C_1$ à $C_4$) -amino, sulfonyle, alkylsulfonyle en $C_1$ à $C_4$, sulfinyle et alkylsulfinyle en $C_1$ à $C_4$ ; alcényle en $C_2$ à $C_7$ qui est non substitué ou qui est substitué avec 1 à 3 atomes d'halogène ; alcynyle en $C_2$ à $C_7$ ; aryle contenant un noyau ayant au moins 6 atomes de carbone ou bien deux noyaux ayant au total dix atomes de carbone et avec des doubles liaisons alternées (liaisons de résonance) entre les atomes de carbone, ledit groupe aryle étant non substitué ou étant substitué avec un ou deux substituants choisis entre des substituants halogéno, alkyle en $C_1$ à $C_4$, trifluorométhyle, cyano, nitro, hydroxy, alkoxy en $C_1$ à $C_4$, (alkyle en $C_1$ à $C_4$)-oxycarbonyle, thiol, alkylthio en $C_1$ à $C_4$, amino, *N*-(alkyle en $C_1$ à $C_4$) amino, *N,N*-di(alkyle en $C_1$ à $C_4$) -amino, sulfonyle, alkylsulfonyle en $C_1$ à $C_4$, sulfinyle et alkylsulfinyle en $C_1$ à $C_4$ ; un système de noyau hétéroaryle en $C_4$ à $C_{12}$ monocyclique ou bicyclique qui est aromatique ou partiellement aromatique, contenant au moins un hétéroatome choisi entre l'oxygène, le soufre et l'azote, ledit système de noyau hétéroaryle en $C_4$ à $C_{12}$ monocyclique ou bicyclique étant non substitué ou étant substitué avec un ou deux substituants choisis entre des substituants halogéno, alkyle en $C_1$ à $C_4$, trifluorométhyle, cyano, nitro, hydroxy, alkoxy en $C_1$ à $C_4$, (alkyle en $C_1$ à $C_4$) oxycarbonyle, thiol, alkylthio en

$C_1$ à $C_4$, amino, *N*-(alkyle en $C_1$ à $C_4$)-amino, *N,N*-di(alkyle en $C_1$ à $C_4$)-amino, sulfonyle, alkylsulfonyle en $C_1$ à $C_4$, sulfinyle et alkylsulfinyle en $C_1$ à $C_4$ ; un groupe hétérocyclique penta- ou hexagonal contenant au moins un hétéroatome choisi entre l'oxygène, le soufre et l'azote, ledit groupe hétérocyclique penta- ou hexagonal étant totalement saturé ou partiellement insaturé et étant non substitué ou étant substitué avec un ou deux substituants choisis entre des substituants halogéno, alkyle en $C_1$ à $C_4$, trifluorométhyle, cyano, nitro, hydroxy, alkoxy en $C_1$ à $C_4$, (alkyle en $C_1$ à $C_4$)oxycarbonyle, thiol, alkylthio en $C_1$ à $C_4$, amino, *N*-(alkyle en $C_1$ à $C_4$)-amino, *N,N*-di-(alkyle en $C_1$ à $C_4$)-amino, sulfonyle, alkylsulfonyle en $C_1$ à $C_4$, sulfinyle et alkylsulfinyle en $C_1$ à $C_4$ ; hydroxy ; hydroxyalcényle en $C_2$ à $C_7$ ; hydroxyalcynyle en $C_2$ à $C_7$ ; alkoxy en $C_1$ à $C_7$ ; thiol ; thioalcényle en $C_2$ à $C_7$ ; thioalcynyle en $C_2$ à $C_7$ ; alkylthiol en $C_1$ à $C_7$ ; amino ; N- (alkyle en $C_1$ à $C_7$) amino ; N,N-di-(alkyle en $C_1$ à $C_7$) amino ; alkylamino en $C_1$ à $C_7$ ; aminoalcényle en $C_2$ à $C_7$ ; aminoalcynyle en $C_2$ à $C_7$ ; aminoalkoxy en $C_1$ à $C_7$ ; alcanoyle en $C_1$ à $C_7$ ; aroyle ; oxoalkyle en $C_1$ à $C_7$ ; alcanoyloxy en $C_1$ à $C_7$ ; carboxy ; (alkyle en $C_1$ à $C_7$) oxycarbonyle, dans lequel le groupe alkyle répond à la définition précitée ; aryloxycarbonyle, dans lequel le groupe aryle répond à la définition précitée ; carbamoyle ; N-(alkyle en $C_1$ à $C_7$) carbamoyle ; N,N-di(alkyle en $C_1$ à $C_7$) carbamoyle ; cyano ; cyano-(alkyle en $C_1$ à $C_7$) ; sulfonyle ; alkylsulfonyle en $C_1$ à $C_7$ ; sulfinyle ; alkylsulfinyle en $C_1$ à $C_7$ ; nitro ; ou un substituant de formule II, dans laquelle les symboles A et m répondent aux définitions précitées ;

et de ses sels et produits de solvatation pharmacologiquement acceptables,
ledit procédé de préparation étant **caractérisé en ce qu'**il comprend :

a) une réaction de condensation d'un alcool de formule IIIb

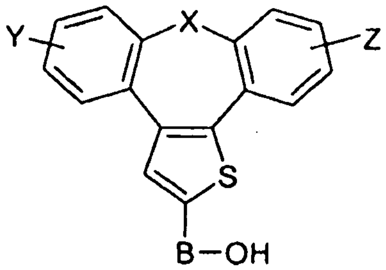

**IIIb**

dans laquelle B représente individuellement un groupe -C(O)-ou $-CH_2-$ et Y et Z répondent aux définitions précitées,
avec un composé de formule IVa

$L^1$ $-(CH_2)_m$ -A **IVa**

dans laquelle $L^1$ représente un groupe partant et m et A répondent aux définitions précitées ; ou
b) une réaction de condensation d'un composé de formule IIIc

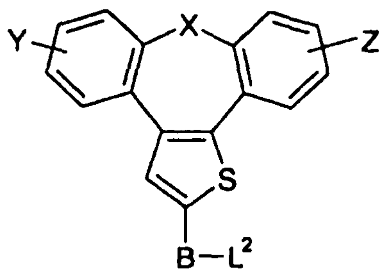

**IIIc**

dans laquelle B représente individuellement un groupe -C(O)-ou $-CH_2-$, $L^2$ représente individuellement un groupe partant et Y et Z répondent aux définitions précitées,
avec un composé de formule IVb

$$HO-(CH_2)_m-A \qquad \textbf{IVb}$$

dans laquelle A et m répondent aux définitions précitées.

**7.** Utilisation d'un composé suivant l'une quelconque des revendications 1 à 5 dans la préparation d'un médicament destiné à être utilisé dans la thérapie de maladies et d'affections inflammatoires induites par la production importante de TNF-α et/ou de IL-1.

**8.** Utilisation suivant la revendication 7, dans laquelle les maladies et affections inflammatoires induites par la production importante de TNF-α et/ou de IL-1 sont choisies dans un groupe consistant en la polyarthrite rhumatoïde, la spondylite rhumatoïde, l'arthrose, les eczémas, le psoriasis, les brûlures provoquées par le rayonnement UV, des maladies ophtalmiques inflammatoires, la maladie de Crohn, la colite ulcérative et l'asthme.

**9.** Formulation pharmaceutique comprenant un composé suivant l'une quelconque des revendications 1 à 5 et un support ou solvant pharmaceutiquement acceptable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3711489 A
- US 4198421 A
- CA 967573
- HR 20020453 A
- WO 01878990 A
- WO 0187890 A

### Non-patent literature cited in the description


- **Cagniant PG ; C. R. Hebd.** *Sceances Acad. Sci.,* 1976, vol. 283, 683-686
- **Carswell EA et al.** *Proc. Natl. Acad. Sci. U. S.A.,* 1975, vol. 72, 3666-3670
- **Elliott M et al.** *Lancet,* 1994, vol. 344, 1105-1110
- **Mori L et al.** *J. Immunol.,* 1996, vol. 157, 3178-3182
- **Pfeffer K et al.** *Cell,* 1993, vol. 73, 457-467
- **Georgopoulos S et al.** *J.Inflamm.,* 1996, vol. 46, 86-97
- **Keffer J et al.** *EMBO J.,* vol. 10, 4025-4031
- **Crohn's.** *Exp. Opin. Invest. Drugs,* 2000, vol. 9, 103
- **Dinarello CA et al.** *Rev. Infect. Disease,* 1984, vol. 6, 51
- **Dinarello CA.** *J. Clinical Immunology,* 1985, vol. 5, 287
- **Bresnihan B et al.** *Arthrit. Rheum.,* 1996, vol. 39, 73
- **Menozzi G.** *J. Heterocyclic Chem.,* 1997, vol. 34, 963-968
- **Jones DC et al.** *J. Med Chem.,* 1984, vol. 27, 1057-1066
- **Green TW ; Wuts PGH.** Protective Groups in Organic Synthesis. John Wiley and Sons, 1999
- **Collier HOJ et al.** *Pharmac. Chemother.,* 1968, vol. 32, 295-310
- **Fukawa K et al.** *J. Pharmacol. Meth.,* 1980, vol. 4, 251-259
- **Schweizer A et al.** *Agents Actions,* 1988, vol. 23, 29-31